# EUROPEAN PATENT SPECIFICATION

(11) **EP 4 514 921 B1**
(45) Date of publication and mention of the grant of the patent: **21.01.2026**
(21) Application number: 23723523.9
(22) Date of filing: 28.04.2023
(51) Int. Cl.: C10G 1/00, C07C 1/20, C07C 1/24, C07C 7/00, C07C 15/46, C07C 407/00, C07C 409/08, C07D 301/19, B01D 3/14, C07C 7/04, C07C 29/132, C10G 1/10

(54) **METHOD FOR STYRENE MONOMER PRODUCTION**
VERFAHREN ZUR HERSTELLUNG VON STYROLMONOMEREN
PROCÉDÉ DE PRODUCTION DE MONOMÈRES DE STYRÈNE

(30) Priority: 29.04.2022 EP 22382419
(43) Date of publication of application: 05.03.2025
(73) Proprietor: Repsol, S.A., 28045 Madrid (ES)
(72) Inventor: PÉREZ VALENCIA, Juan Pedro, 28935 Móstoles (ES); ENCINAS ALÍAS, Ignacio, 28935 Móstoles (ES)
(74) Representative: ZBM Patents - Zea, Barlocci & Markvardsen
(86) International application number: PCT/EP2023/061305
(87) International publication number: WO 2023/209180

(56) References cited:
- WO-A1-2021/180893
- US-A1- 2018 312 764
- US-A1- 2022 098 490

## Description

The present invention relates to a process for the incorporation of products originating from waste plastic feedstocks into a propylene oxide styrene monomer co-production process.

### Background art

US 2022/098490 A1 discloses a generic process for producing chemicals from waste plastic. In some embodiment it discloses converting petroleum and pyrolysis oil into styrene, the process involving the use of ethylbenzene. WO 2021/180893 A1 discloses a process for recovering styrene monomer from a plastic material comprising pyrolysis of said plastic material and separation steps. US 2018/312764 A1 discloses upgrading of an organic stream comprising oxygenates. The process involves hydrotreatment and separation of ethylbenzene by distillation.

Co-production of propylene oxide (PO) and styrene monomer (SM), also referred to as the "PO/SM" process, involves the oxidation of ethylbenzene (EB) to form ethylbenzene hydroperoxide (EBHP), followed by the catalytic reaction of the EBHP with propylene to produce PO and 1-phenylethanol (MBA, also referred as alpha-methylbenzyl alcohol), PO purification, dehydration of the MBA to produce SM; and hydrogenation of methyl phenyl ketone (MPK), which is side-product of the first two reactions, to obtain MBA, for subsequent conversion to SM.

The PO/SM process requires a rigorous control of the specifications of the different stream flows, as well as of the working conditions at each stage of the process. The presence in the different stages of the PO/SM process of different contaminants may cause several problems such as the contamination of the styrene monomer product with different co-boilers such as xylenes; the presence of light molecules such as alkanes, alkenes, cycloalkanes, etc cause impurification of PO product; the presence of insaturated molecules in the EB containing stream may cause the formation of polymers that foul and clog the process; the presence of acids in the recycle EB results in a worsening of the oxidation selectivity.

Recycling of waste plastics has been a topic of interest in the fields of environmental science and technology for some time.

It is well known that waste plastic can be pyrolyzed to produce high yields of light gas olefins (i.e., ethylene, propylene, butylenes) and aromatics (i.e., benzene, toluene, xylenes (BTX), ethylbenzene (EB)), along with low yields of paraffins, isoparaffins, and naphthenes. The pyrolysis can be configured to maximize propylene and/or aromatics, with high yields of BTX and EB.

There have been disclosed in the art, processes to recycle polystyrene by pyrolytic depolymerization of a styrene-containing plastics waste. A major disadvantage is that although styrene yields may be high, also significant amounts of by-products are formed, thus resulting in a SM product which does not fulfill international market specifications (e.g. ASTM D-2827). Thus, polystyrene may be reproduced from liquid fractions of pyrolysis oil of polystyrene but with inferior properties compared to a polystyrene prepared from neat styrene (pure styrene).

The challenges of purification of styrene monomer (SM) contained in pyrolysis oil are the presence of molecules with relative volatility similar to SM (o-xylene, p-xylene, m-xylene...), whose reduction to the required specification levels is not feasible by conventional rectification and, molecules with close relative volatilities (EB, Cumene, n-propylbenzene...), which require a very high number of separation steps by distillation and/or amounts of energy. Additionally, there are molecules with heteroatoms (S, N, O, halogens) and products that induce coloration.

Thus, there is an ongoing need to develop methos for producing styrene monomer from feedstocks other than crude oil, for example from feedstocks derived from waste plastic, but without jeopardize the global process.

### Summary

Inventors have surprisingly found that a hydrocarbon liquid stream resulting from the pyrolysis of waste plastic, such as from pyrolysis oil of waste plastics, comprising polystyrene, is appropriate for incorporation in the propylene oxide (PO) and styrene monomer (SM) co-production process, without compromising overall performance of the process, as well as properties and quality of the different streams and final products thus obtained.

As it is disclosed in the state of the art, the oils obtained from the pyrolysis of waste plastics are a complex mixture formed by a lot of compounds that can be potentially incompatibles with the different stages of the PO/SM process. The hydrocarbon liquid stream resulting from the pyrolysis of waste plastics contains components considered impurities in the final products and components which affect the chemical reactions which occurs in the PO/SM process, making the product theoretically incompatible with the PO/SM process.

Nevertheless, contrary to the knowledge of the state of the art, as it is demonstrated in the experimental section, the present invention discloses that the use of a hydrocarbon liquid stream (also refered herein as pyrolysis oil) obtained from a polystyrene enriched in styrene monomer waste plastic may be incorporated into the PO/SM process, without jeopardize neither the global performance of the process nor the quality and properties of the products finally obtained. The method allows reducing the synthesis de novo of styrene monomer and offers means for polystyrene waste treatment different from incineration and/or dump.

Furthermore, the hydrocarbon liquid stream obtained from a polystyrenic-based waste plastics can be used in already existing equipment for the PO/SM coproduction process, without being modified, because it is compatible with the commonly used equipment without dirtying or ruining the equipment.

Then, the use of a hydrocarbon liquid stream obtained from the pyrolysis of a polystyrene enriched in styrene monomer waste plastics is advantageous because allows preparing efficiently circular styrene monomer using an oil, which does not require to perform costly and tedious fractionating and/or purifying steps. Additionally, the use of a hydrocarbon liquid stream obtained from the pyrolysis of polystyrene enriched in styrene monomer waste plastics results in the recovery of styrene monomer and ethylbenzene present in the pyrolysis oil, improving the global yield when compared with purification processes which only recover styrene monomer.

Disclosed herein are processes for producing high value products such as styrene monomer by processing waste plastic. The process may include conversion of waste plastic, which can be cracked or pyrolyzed by means of low temperature or high temperature pyrolysis, and by thermal or catalytic pyrolysis, wherein the hydrocarbon liquid product thus obtained may be incorporated to the PO/SM process under particular conditions.

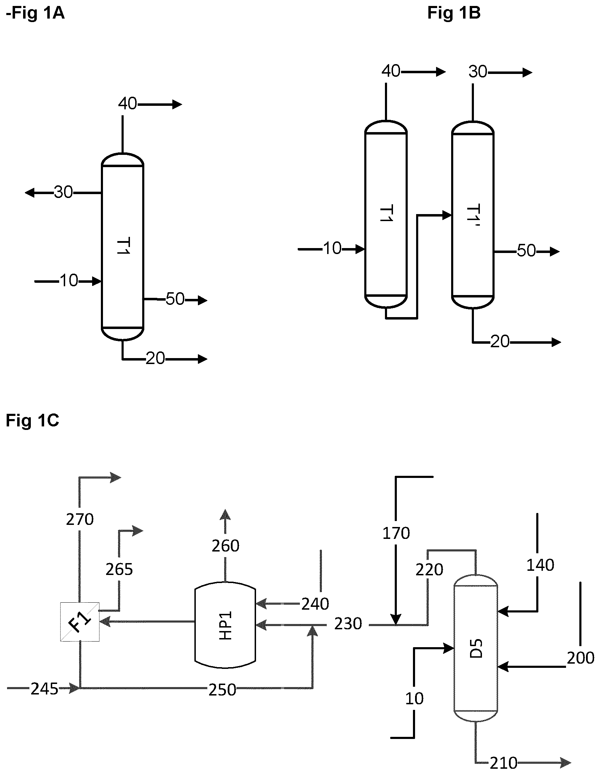

Thus, a first aspect of the present invention relates to a method comprising:
a) providing a hydrocarbon liquid stream (10) resulting from the pyrolysis of waste plastic;
b) introducing the hydrocarbon liquid stream (10) into
   b.1) a first fractional distillation unit (T1) to separate a C7 stream overhead (40), a first fractionated sidestream (30), a heavies-containing stream (20) from bottom, and, optionally, an alpha-methyl-styrene containing product sidestream (50) (see Fig 1A);
      alternatively,
   b.2) a first fractional distillation unit (T1) to separate a C7 stream overhead (40) and a stream which is introduced into a second fractional distillation unit (T1') obtaining a first fractionated stream (30), a heavies containing stream (20) from bottom, and, optionally, an alpha-methyl-styrene containing product sidestream (50) (see Fig 1B);
      or alternatively
   b.3) a distillation column (D5), thus obtaining a distilled out product stream (220); (see Fig 1C);
c) feeding
   c.1) the first fractionated sidestream (30) obtained in b.1) or b.2), or the distilled out product stream (220) obtained in b.3), to a hydroprocessing unit (HP) comprising a heterogeneous hydroprocessing catalyst in the presence of hydrogen, to yield a ethylbenzene containing product stream (80, 270);
      or alternatively,
   c.2) the first fractionated sidestream (30) obtained in b.1) or b.2), to a distillation column (D5), obtaining a distilled out product stream (220); the distilled out product stream (220) is then fed to the hydroprocesing unit (HP) comprising a heterogeneous hydroprocessing catalyst in the presence of hydrogen to yield a ethylbenzene containing product stream (270);
d) incorporating the ethylbenzene containing product stream (80, 270) thus obtained in c.1) or c.2), to an ethylbenzene distillation column (D1), thus a distilled ethylbenzene stream (110) is recovered from the top of the tower and an alpha-methylbenzyl alcohol containing stream (120) is recovered from bottoms;
e) incorporating the ethylbenzene product stream (110), optionally together with fresh ethylbenzene (100), to a ethylbenzene oxidation unit to produce ethylbenzene hydroperoxide by oxidation in liquid phase;
f) reacting the ethylbenzene hydroperoxide obtained in e) with a propylene stream in a epoxidation unit in the presence of a catalyst to produce crude propylene oxide (300) and a bottoms product (310) containing ethylbenzene and alpha-methylbenzyl alcohol;
g) feeding the alpha-methylbenzyl alcohol containing stream (120) to a second distillation column (D2), thus obtaining three fractions: a xylene containing product stream overhead (130), a alpha-methylbenzyl alcohol rich sidestream (150), and a bottom stream (140) containing alpha-methylbenzyl alcohol and higher boiling point products;
   or alternatively, the alpha-methylbenzyl alcohol containing stream (120) is introduced into a second distillation column (D2) to separate a xylene containing stream product overhead (130), and a bottom stream which is introduced into an additional distillation column (D2'), thus obtaining a alpha-methylbenzyl alcohol rich top stream (150), and a heavy bottom stream (140) containing alpha-methylbenzyl alcohol and higher boiling point products;
h) incorporating the alpha-methylbenzyl alcohol rich sidestream (150) to a dehydration unit (DH1) containing a catalyst, thus obtaining crude styrene monomer (160) and water, which are phase separated;
i) feeding the crude styrene phase (160) thus obtained to a distillation column (D3) wherein a ethylbenzene containing stream (170) is top separated, and the bottom product (180) is fed to a styrene product column (D4) where styrene monomer is obtained at the top (190), and a heavy product at the bottom (200);
j) feeding the heavy bottom product streams (140 and 200), resulting from the second and the additional distillation columns (D2, D2') and styrene product column (D4), to the distillation column (D5); the distilled out product stream (220) thus obtained is fed alongside the ethylbenzene containing stream (170) to the hydroprocesing unit (HP) of step c).

Steps d) to j) corresponds to current Propylene Oxide / Styrene Monomer (PO/SM) co-production process as described in the prior art (see for example Propylene Oxide PERP 2012-7 pages 19 - 26). Details are given in the detailed description in order to follow the implications of incorporating a hydrocarbon liquid stream resulting from the pyrolysis of waste plastic as described above in step c).

### Brief description of the drawings

Non-limiting examples of the present disclosure will be described in the following, with reference to the appended drawings, in which:
Fig. 1A depicts a schematic representation of step b.1)
Fig 1B depicts a schematic representation of step b.2)
Fig 1C depicts a schematic representation of step b.3)
Fig 2A depicts a schematic representation of step c.1A)
Fig 2B depicts a schematic representation of general PO/SM process incorporating step b.1) + c.1A).
Fig 3A depicts a schematic representation of step c.1B)
Fig 3B depicts a schematic representation of general PO/SM process incorporating step b.1) + c.1B) - Example 1 according to the invention.
Fig 4A depicts a schematic representation of step c.2)
Fig 4B depicts a schematic representation of general PO/SM process incorporating step b.1) + c.2) - Example 2 according to the invention.
Fig 5A depicts a schematic representation of MBA distillation step using one distillation column D2
Fig 5B depicts a schematic representation of MBA distillation step using two distillation columns D2 + D2'
Fig 6 depicts a schematic representation of general PO/SM process (comparative example 1)
Fig 7 depicts a schematic representation of comparative example 2
Fig 8 depicts a schematic representation of comparative example 3

### Detailed description

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

For the purposes of the present invention, any ranges given include both the lower and the upper end-points of the range. Ranges and values given, such as temperatures, times, and the like, should be considered approximate, unless specifically stated.

The terms "percentage (%) by weight" or "percentage (%) w/w" or "%wt" have the same meaning and are used interchangeable. This term refers to the percentage of a component in relation to the total weight.

For purposes of the disclosure herein, the term "amount" refers to a weight % of a given component in a particular composition, based upon the total weight of that particular composition (e.g., the total weight of all components present in that particular composition), unless otherwise indicated.

The terms "a," "an," and "the" do not denote a limitation of quantity, but rather denote the presence of at least one of the referenced item. As used herein the singular forms "a," "an," and "the" include plural referents.

The term "about" or "around" as used herein refers to a range of values ± 10% of a specified value. For example, the expression "about 25" or "around 25" includes ± 10% of 25, i.e. from 22.5 to 27.5.

For the purpose of the invention the terms methyl-styrene, ethyl-styrene and xylene encompasses the ortho-, meta-, and para- isomers, unless specifically described. The terms of alkanes, alkenes and alkynes that are indicated in plural in the present invention indicate that they encompass all the possible regio- and stereoisomers thereof. For example the term pentenes encompass 1-pentene, 2-cis-pentene, and 2-trans-pentene; and the term methyl butene encompasses 2-methyl-2-butene, 2-methyl-1-butene and 3-methyl-1-butene.

For the purpose of the invention, the expressions "obtainable", "obtained" and equivalent expressions are used interchangeably, and in any case, the expression "obtainable" encompasses the expression "obtained".

Further, for the purpose of the invention, wherein the stereo- and regio-selectivity of a term (compound) is not specifically disclosed, it is understood that this term encompasses all stereo- and regio-isomers. Then, for the purpose of the invention, the term "methyl-styrene" encompasses 1-methyl-2-vinylbenzene (ortho-methyl-styrene), 1-methyl-3-vinylbenzene (meta-methyl-styrene), 1-methyl-4-vinylbenzene (para-methyl-styrene),

The term "polystyrene" refers to a polymer comprising the styrene monomer (SM; or C6H5CH=CH2) of formula:

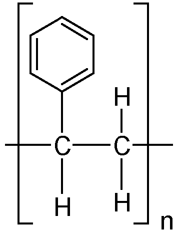

The term "polystyrene" encompasses both homopolymers of styrene monomer and copolymers of styrene monomers with other monomers, thus for the purpose of the invention, the term polystyrene encompasses, in all their grades, pure polystyrene, styrene copolymers, and mixture thereof. In an embodiment, the polystyrene is a residue (waste) containing polystyrene. Examples of suitable polymers containing styrene for the present invention includes, but it is not limited to, High Impact Polystyrene (HIPS), styrene acrylonitrile (SAN), Acrylonitrile Butadiene Styrene (ABS), General Purpose polystyrene (GPPS), Expanded polystyrene foam (EPS).

The term "alkane" and "cycloalkane" refers to a saturated straight hydrocarbon, branched, linear or cycle hydrocarbon optionally branched, which contains the number of carbon atoms specified in the description or claims. Examples include methane, ethane , n-butane, isobutane, pentane, hexane, methylbutane, heptane and, octane and cyclopentane.

The term "alkene" refers to a straight hydrocarbon, optionally branched, which contains the number of carbon atoms specified in the description or claims, and that also contains at least one carbon-carbon double bond. Examples include, among others, the ethenyl, 2-propenyl, and 1-propenyl. Examples include 2-methyl-1-butene, pentene, heptene, hexene, and isobutene.

The term "cycloalkene" refers to a cycle hydrocarbon, optionally branched, which contains the number of carbon atoms specified in the description or claims, and that also contains at least one carbon-carbon double bond. Examples include cyclopentene, cyclohexene, methylcyclohexene, dimethylcyclohexene, 1,3-dimethylcyclopentadiene and cyclopentadiene.

The terms of alkanes, alkenes and alkynes that are indicated in plural in the present invention indicate that they encompass all the possible regio- and stereoisomers thereof. For example, the term "pentenes" encompass 1-pentene, 2-cis-pentene, and 2-trans-pentene; and the term methyl butene encompasses 2-methyl-2-butene, 2-methyl-1-butene and 3-methyl-1-butene.

The term "aromatic compound" refers to those organic compounds that contain one or more rings with pi electrons delocalized all the way around them which contains the number of carbon atoms specified in the description or claims. The aromatic compounds can be optionally substituted by one or more moieties such as for examples (C1-C6)alkyl, (C2-C6)alkenyl, (C2-C6)alkynyl. In an embodiment, the aromatic compound is a phenyl optionally substituted by (C1-C6)alkyl, (C2-C6)alkenyl, (C2-C6)alkynyl. The term "alkyl" refers to a saturated straight hydrocarbon substituent, optionally branched, which contains the number of carbon atoms specified in the description or claims. Examples include, among others, the group methyl, ethyl, propyl, isopropyl, butyl, isobutyl, sec-butyl, and tert-butyl. The term "alkenyl" refers to straight hydrocarbon substituent, optionally branched, which contains the number of carbon atoms specified in the description or claims, and that also contains at least one carbon-carbon double bond.

The term "alkynyl" refers to straight hydrocarbon substituent, optionally branched, which contains the number of carbon atoms specified in the description or claims, and that also contains at least one carbon-carbon triple bond.

In an embodiment, the aromatic compounds are selected from the group consisting of C8 aromatic compounds, C9 aromatic compounds and C10 aromatic compounds. Examples of C8 aromatic compounds include ethylbenzene; ortho-xylene, meta-xylene, and para-xylene; phenylacetylene. Examples of C9 aromatic compounds include cumene, propenyl benzene, n-propyl benzene, allyl-benzene, ethyl-toluene, and methyl-styrene. Examples of C10 aromatic compounds include divinylbenzene and ethyl-styrene.

The term "heavy oil fraction" or "heavies containing stream" refers to a mixture with an initial normal boiling point of 200°C and final normal boiling point of 400°C. It encompasses one or more of the following compounds indene, naphthalene, phthalene, tetrahydronaphthalene, 2,4-diphenyl-1-butene, 1,4-diphenyl-1,3-butadiene, 2,4,6-triphenyl-1-hexene, biphenyl, bibenzyl, methyl naphthalene, 2-phenylnaphtalene, diphenylmethane, dimers, trimers, stilbene among others. In general terms, "heavies containig stream (20) may also be referred as a C9+ containing stream.

The term "acid containing compounds" refers to compounds comprising one or more acids moieties. It encompasses both organic carboxyl acids and inorganic acids. Examples include, among others, hydrochloric acid, hydrobromic acid, benzoic acid, acetic acid and propionic acid.

The term "metal" encompasses for instance calcium, Potassium, silicium, aluminium, sodium, magnesium, titanium, iron, zinc, among others.

The term "heteroatom" encompasses halogen (for instance chlorine, fluorine, and bromine), sulphur, nitrogen and oxygen. And the term "heteroatom containing compounds" encompasses compounds that comprises at least one or more heteroatoms as defined above.

For the purpose of the invention, the terms "hydrocarbon liquid stream obtained from a polystyrenic-based waste plastic", "pyrolysis oil of polystyrene", "polystyrene pyrolysis oil" and "pyrolysis oil" have the same meaning and are used interchangeable. They refer to an oil mixture containing from 50% to 98% of styrene monomer obtainable by the pyrolysis of polystyrene as defined above. Pyrolysis is a well-established technique for decomposition of organic material into oil and other constituents at elevated temperatures (particularly, above its decomposition temperature), in an inert atmosphere (in the absence of oxygen). Appropriate pyrolysis oils for the present invention and processes for their preparation are widely disclosed in the state of the art. Examples can be found in Ki-bum Park et al. "Two-stage pyrolysis of polystyrene: Pyrolysis oil as a source of fuel or benzene, toluene, ethylbenzene, and xylenes". Applied Energy 259, 2020, pp. 114240; Ibrahim M. Maafa. "Pyrolysis of Polystyrene Waste: A review". Polymers, 2021, vol. 13, pp. 225.; John Scheirs, and Walter Kaminsky. "Feedstock Recycling and Pyrolysis of Waste Plastics: Converting Waste Plastics into Diesel and Other Fuels", Wiley Online Library 2006 p. 635 ; and Jasmin Shah et al. "Conversion of waste polystyrene through catalytic degradation into valuable products", Korean J. Chem. Eng., 2014, vol. 31(8), pp.1389-1398, US10301235, WO2021230312, and WO2021053074.

In accordance with one embodiment, optionally in combination with one or more features of the particular embodiments defined herein, the polystyrene pyrolysis oil comprises from 50% to 98% by weight of styrene monomer by submitting one or more polystyrene compounds and/or one or more polystyrene containing compounds, particularly a polystyrene enriched in styrene monomer, to a pyrolysis reaction (for instance see US10301235, WO2021230312 and WO2021053074).

In accordance with an embodiment, optionally in combination with one or more features of the particular embodiments defined herein, the hydrocarbon liquid stream resulting from the pyrolysis of waste plastic is a pyrolysis oil stream resulting from the conversion of a waste plastic, preferably a polystyrenic rich waste plastic, to a pyrolysis oil stream and a solid residue (char) in a pyrolysis unit.

In accordance with another embodiment, optionally in combination with one or more features of the particular embodiments defined herein, the process is one wherein the hydrocarbon liquid stream (10) is a pyrolysis oil comprising
- from 50 to 98% by weight of styrene monomer plus ethylbenzene, preferably from 60 to 98% by weight, more preferably from 70 to 98% by weight, even more preferably from 80 to 98% by weight;
- from 0 to 9% by weight of alpha-methyl styrene, preferably from 0.01 to 8% by weight, more preferably from 0.1 to 5% by weight, even more preferably from 0.1 to 2% by weight;
- from 0 to 1% by weight of phenylacetylene, preferably from 0.001 to 1% by weight, more preferably from 0.01 to 1% by weight, even more preferably from 0.1 to 1% by weight;
- from 0 to 15% by weight of toluene, preferably from 1 to 12% by weight, more preferably from 2 to 10% by weight, even more preferably from 2 to 8% by weight;
- from 0 to 5% by weight of benzene, preferably from 0,1 to 4% by weight, more preferably from 0,5 to 4% by weight, even more preferably from 0,5 to 2% by weight;
- from 0 to 2.5% by weight of C8 aromatic compounds others than styrene monomer and ethylbenzene, a mixture of; ortho-xylene, meta-xylene, and para-xylene, preferably from 0 to 2% by weight, more preferably from 0.1 to 1.5% by weight, even more preferably from 0.1 to 1% by weight;
- from 0 to 5% by weight of C9 aromatic compounds others than alpha-methyl styrene a mixture of cumene; beta-methyl-styrene, n-propylbenzene; allyl-benzene, ethyl-toluene and methyl-styrene, preferably from 0.001 to 3% by weight, more preferably from 0.01 to 2% by weight, even more preferably from 0.1 to 1% by weight;
- from 0 to 5% by weight of C10 aromatic compounds a mixture of divinylbenzene and ethyl-styrene, preferably from 0.001 to 3% by weight, more preferably from 0.01 to 2% by weight, even more preferably from 0.1 to 1% by weight;
- from 0 to 2% by weight of one or more compounds selected form the group consisting of a mixture of (C3-C8) alkanes, (C3-C8)alkenes, (C5-C8)cycloalkanes and (C5-C8)cycloalkenes, preferably from 0.001 to 1.5% by weight, more preferably from 0.01 to 1% by weight, even more preferably from 0.1 to 0.8% by weight;
- from 0.05 to 15% by weight of a heavy oil fraction, preferably from 0.1 to 10% by weight, more preferably from 0.5 to 8% by weight, even more preferably from 1 to 5% by weight;
- from 0 to 50 ppm by weight of metals, of metal containing compounds, preferably from 1 to 25 ppm by weight, more preferably from 5 to 10 ppm by weight;
- from 0.5 to 5000ppm by weight of heteroatom, of heteroatom containing compounds , preferably from 1 to 2500ppm by weight, more preferably from 5 to 100ppm by weight, even more preferably from 10 to 500ppm by weight; and
- from 0 to 1 % by weight water, preferable from 0.001 to 0.1 % by weight, more preferably from 0.005 to 0.05 % by weight, even more preferably 0.01 to 0.025 % by weight.

In accordance with another embodiment, optionally in combination with one or more features of the particular embodiments defined herein, the process is one wherein the hydrocarbon liquid stream (10) is a pyrolysis oil comprising:
- from 50 to 98% by weight of styrene monomer plus ethylbenzene;
- from 0 to 9% by weight of alpha-methyl styrene;
- from 0 to 1% by weight of phenylacetylene;
- from 0 to 15% by weight of toluene;
- from 0 to 5% by weight of benzene;
- from 0 to 2.5% by weight of C8 aromatic compounds others than styrene monomer and ethylbenzene comprising; ortho-xylene, meta-xylene, and para-xylene;
- from 0 to 5% by weight of C9 aromatic compounds others than alpha-methyl styrene comprising cumene; beta-methyl-styrene, n-propylbenzene; allyl-benzene, ethyl-toluene, and methyl-styrene;
- from 0 to 5% by weight of C10 aromatic compounds comprising divinylbenzene and ethyl-styrene;
- from 0 to 2% by weight of one or more compounds selected form the group consisting of (C3-C8) alkanes, (C2-C8) alkenes, (C5-C8)cycloalkanes and (C5-C8)cycloalkenes;
- from 0.05 to 15% by weight of a heavy oil fraction;
- from 0 to 50 ppm by weight of metals, of metal containing compounds;
- from 0.5 to 5000ppm of heteroatom, of heteroatom containing compounds;
   and
- from 0 to 1 % by weight of water.

In accordance with another embodiment, optionally in combination with one or more features of the particular embodiments defined herein, the process is one wherein the hydrocarbon liquid stream (10) is a pyrolysis oil comprising:
- from 60 to 98% by weight of styrene monomer plus ethylbenzene;
- from 0.1 to 5% by weight of alpha-methyl styrene;
- from 0.01 to 1% by weight of phenylacetylene;
- from 2 to 10% by weight of toluene;
- from 0.5 to 4% by weight of benzene;
- from 0.1 to 1.5% by weight of C8 aromatic compounds; particularly comprising ethylbenzene; ortho-xylene, meta-xylene, and para-xylene;
- from 0.01 to 2% by weight of C9 aromatic compounds; particularly comprising cumene, propenylbenzene, n-propylbenzene, allyl-benzene, ethyl-toluene, and methyl-styrene
- from 0.01 to 2% by weight of C10 aromatic compounds; particularly comprising divinylbenzene and ethyl-styrene;
- from 0.01 to 1% by weight of one or more compounds selected form the group consisting of (C3-C8) alkanes, (C2-C8) alkenes, (C5-C8)cycloalkanes and (C5-C8)cycloalkenes;
- from 0.5 to 8% by weight of a heavy oil fraction;
- from 5 to 10 ppm by weight of metals, of metal containing compounds;
- from 10 to 500ppm of heteroatom, of heteroatom containing compounds; and
- from 0.01 to 0.025% by weight water.

In accordance with another embodiment, optionally in combination with one or more features of the particular embodiments defined herein, the process is one wherein the hydrocarbon liquid stream (10) is a pyrolysis oil as defined above further comprises from 0.005 to 3 mgKOH/g of acid containing compounds measured by the standard test method ASTM D664, preferably 0.01 to 1 mgKOH/g of acid containing compounds.

In accordance with another embodiment, optionally in combination with one or more features of the particular embodiments defined herein, the process is one wherein the hydrocarbon liquid stream (10) is a pyrolysis oil as defined above comprising from 0.1 to 50 ppm of metals, preferably from 0.1 to 5 ppm measured by the method EPA 3051a and ASTM D6052-97.

The term "heavy oil fraction" refers to a mixture with an initial normal boiling point of 200°C and final normal boiling point of 400°C. It encompasses one or more of the following compounds indene, naphthalene, tetrahydronaphthalene, 2,4-diphenyl-1-butene, 1,4-diphenyl-1,3-butadiene, 2,4,6-triphenyl-1-hexene, biphenyl, bibenzyl, methyl naphthalene, 2-phenylnaphtalene, diphenylmethane, and stilbene among others.

The term "acid containing compounds" refers to compounds comprising one or more acids moieties. It encompasses both organic carboxyl acids and inorganic acids. Examples include, among others, hydrochloric acid, hydrobromic acid, benzoic acid, acetic acid, and propionic acid.

The term "metal" encompasses for instance calcium, potassium, silicium, aluminium, sodium, magnesium, titanium, iron, zinc, among others.

Any hydrocarbon liquid stream resulting from the treatment of waste plastic, other than a pyrolysis oil stream, comprising a composition as defined above may also be used in the process of the invention.

In accordance with another embodiment, optionally in combination with one or more features of the particular embodiments defined herein, the process further comprises a previous step wherein, in a pyrolysis unit configured to convert the waste plastic, via pyrolysis process, to a pyrolysis product, wherein the pyrolysis product comprises a gas phase and a solid phase (char), and the gas phase is partially condensed in a liquid phase (pyrolysis oil) and a uncondensable gas phase containig pyrolysis gases, such as C1 to C4 hydrocarbons, hydrogen, carbon monoxide, carbon dioxide, hydrochloric acid gas, etc.).

Waste plastic which can be loaded into or fed to the pyrolysis unit may include post-consumer waste plastics, such as mixed waste plastic. Mixed plastics can comprise non-chlorinated plastics (e.g., polyolefins, polyethylene, polypropylene, polystyrene, copolymers, etc.), chlorinated plastics (e.g., polyvinylchloride (PVC), polyvinylidene chloride (PVDC), etc.), and the like, or mixtures thereof. Preferably, waste plastic comprises a high polystyrene content.

The pyrolysis unit may be any suitable vessel configured to convert waste plastics into gas phase and solid phase (char) products (e.g., simultaneously), gas phase is partially condensed in a liquid phase. The vessel may be configured for gas phase, liquid phase, vapor-liquid phase, gas-solid phase, liquid-solid phase, or slurry phase operation. The vessel may contain one or more beds of inert material or pyrolysis catalyst, such as e.g. sand, zeolite, alumina, a catalytic cracking catalyst, or combinations thereof. Generally, the pyrolysis catalyst is capable of transferring heat to the components subjected to the pyrolysis process in the pyrolysis unit. Alternatively, the pyrolysis unit can be operated without any catalyst (e.g., pure thermal pyrolysis). The pyrolysis unit may be operated adiabatically, isothermally, nonadiabatically, non-isothermally, or combinations thereof. The pyrolysis reactions may be carried out in a single stage or in multiple stages. For example, the pyrolysis unit can be two or more reactor vessels fluidly connected in series.

### Step b) - Pretreatment of the hydrocarbon liquid stream (10)

In the process according to the invention, step b) may be carried out according to 3 different alternatives, namely:
b.1) wherein the hydrocarbon liquid stream (10) is introduced into a first fractional distillation unit (T1) - see Fig 1A;
b.2) wherein the hydrocarbon liquid stream (10) is introduced into a first fractional distillation unit (T1) to separate a C7 stream overhead (40) and a stream which is introduced into a second fractional distillation unit (T1') - see Fig 1B;
   or alternatively
b.3) wherein the hydrocarbon liquid stream (10) is introduced into a distillation column (D5), without being previously fractionated in any fractional distillation unit (T1 or T1'), thus obtaining a distilled out product stream (220) - see Fig. 1C.

Thus, in accordance with option b.1), the hydrocarbon liquid stream (10) is introduced into a first fractional distillation unit (T1) to separate a C7 stream overhead (40), a first fractionated sidestream (30), a heavies-containing stream (20) from bottom, and, optionally, an alpha-methyl-styrene containing product second sidestream (50) - see Fig. 1A.

In accordance with option b.2), the hydrocarbon liquid stream (10) is introduced into a first fractional distillation unit (T1) to separate a C7 stream overhead (40) and a stream which is introduced into a second fractional distillation unit (T1') obtaining a first fractionated stream (30), a heavies containing stream (20) from bottom, and, optionally, an alpha-methyl-styrene containing product sidestream (50) - see Fig 1B.

In accordance with option b.3) the hydrocarbon liquid stream (10) is introduced into a distillation column (D5), without being previously fractionated in any fractional distillation unit (T1 or T1'), thus obtaining a distilled out product stream (220); the distilled out product stream (220) is then fed to the hydroprocesing unit (HP) of step c.1) - see Fig 1C.

In accordance with an embodiment, optionally in combination with one or more features of the particular embodiments defined herein, the first fractionated sidestream (30) comprise a benzene content of from 0 to 500 ppmw, preferably from 0 to 250 ppmw.

In accordance with an embodiment, optionally in combination with one or more features of the particular embodiments defined herein, the first fractionated sidestream (30), comprise a styrene and ethylbenzene content from 89 wt% to 99 wt%, preferably from 92 wt% to 99 wt%, more preferably from 95 wt% to 98.9 wt% as the summe of weight content of both components in relation to the total weight of the stream.

In accordance with an embodiment, optionally in combination with one or more features of the particular embodiments defined herein, the first fractionated sidestream (30) obtained following either option b1) or option b2), comprises a styrene and ethylbenzene content from 89 wt% to 99 wt%, preferably from 92 wt% to 99 wt%, more preferably from 95 wt% to 98.9 wt% as the summe of weight content of both components in relation to the total weight of the stream, and a benzene content from 0 to 250 ppmw in relation to the total weight of the stream.

In accordance with an embodiment, optionally in combination with one or more features of the particular embodiments defined herein, the first fractionated sidestream (30) obtained following either option b1) or option b2), comprises from 0.01 to 1 mgKOH/g of acid containing compounds measured by the standard test method ASTM D664.

In accordance with an embodiment, optionally in combination with one or more features of the particular embodiments defined herein, the first fractionated sidestream (30) obtained following either option b1) or option b2), comprises from 0 to 1 ppm F, from 0 to 5 ppm Cl, from 0 to 5 ppm Br and from 0 to 500 ppm N.

In accordance with another embodiment, optionally in combination with one or more features of the particular embodiments defined herein, the first fractionated sidestream (30) obtained following either option b1) or option b2), comprises
i. from 89 to 99 % by weight of styrene monomer plus ethylbenzene, preferably from 90 to 98% by weight, more preferably from 93 to 95% by weight;
ii. from 0 to 500 ppm by weight of benzene, preferably from 50 to 300 ppm by weight, more preferably from 100 to 250 ppm by weight;
iii. from 0 to 1 % by weight of toluene, preferably from 0.01 to 0.5% by weight, more preferably from 0.1 to 0.3% by weight;
iv. from 0 to 2.5 % by weight of xylenes, preferably from 0.1 to 2% by weight, more preferably from 0.5 to 1.5% by weight;
v. from 0 to 1.5 % by weight of C9 aromatic compounds others than alpha-methyl styrene a mixture of cumene; propenylbenzene, n-propylbenzene; allyl-benzene, ethyl-toluene and methyl-styrene, preferably from 0.01 to 1% by weight, more preferably from 0.1 to 0.8% by weight;
vi. from 0 to 5 % by weight of alpha-methyl styrene, preferably from 0.1 to 3% by weight, more preferably from 1 to 2.5% by weight;
vii. from 0 to 1.5 % by weight of phenylacetylene preferably from 0.0001 to 1% by weight, more preferably from 0.001 to 0.5% by weight;
viii. from 0 to 2500 ppm by weight of heteroatom containing compounds , preferably from 1 to 1250 ppm by weight, more preferably from 10 to 250 ppm by weight;
ix. from 0 to 0.1% by weight of C10 aromatic compounds a mixture of divinylbenzene and ethyl-styrene, preferably from 0.001 to 0.08% by weight, more preferably from 0.01 to 0.05% by weight;
x. from 0 to 1% by weight of one or more compounds selected from the group consisting of a mixture of (C3-C8) alkanes, (C2-C8)alkenes, (C5-C8)cycloalkanes and (C5-C8)cycloalkenes, preferably from 0.001 to 0.85% by weight, more preferably from 0.01 to 0.5% by weight;
xi. from 0 to 0.1% by weight of a heavy oil fraction, preferably from 0.0001 to 0.05% by weight, more preferably from 0.001 to 0.01% by weight;
xii. from 0 to 50 ppm by weight of metals, of metal containing compounds, preferably from 0.01 to 25 ppm by weight, more preferably from 0.1 to 10 ppm by weight;
xiii. from 0.5 to 1500 ppm by weight of heteroatom, of heteroatom containing compounds preferably from 1 to 500 ppm by weight, more preferably from 5 to 100 ppm by weight; and
xiv. from 0 to 120 ppm by weight of water, preferably from 0.1 to 25 ppm by weight, more preferably from 1 to 10 ppm by weight.

### Option b.1) - pretreatment of the hydrocarbon liquid stream (10) using a fraction distillation unit (T1) - see Fig 1A

In accordance with an embodiment, option b.1) optionally in combination with one or more features of the particular embodiments defined herein, step b) is carried out in a first fraction distillation unit (T1) to separate a C7 stream overhead (40), a first fractionated sidestream (30), a heavies-containing stream (20) from bottom, and, optionally, an alpha-methyl-styrene containing product second sidestream (50).

In accordance with this embodiment, the first fractional distillation unit (T1) can be a packed distillation column, a trays distillation column or a combination thereof. The column may be operated under vacuum pressure, for example 13 to 65 kPa and a temperature of 180°C in the bottom section, for example, 15 to 50 kPa, for example 20 to 35 kPa. The temperature may be greater than or equal to 90°C, for example, greater than or equal to 130°C, for example, greater than or equal to 175°C.

Top of the column (40) consists on benzene, toluene, water, some of the components belonging to the fed oil fraction (C3-C8) alkanes, (C2-C8)alkenes, (C5-C8)cycloalkanes and (C5-C8)cycloalkenes with higher volatilities compared to styrene monomer, some of the acid and heteroatom containing compounds of the fed oil with higher volatities compared to styrene monomer and, part of the ethylbenzene of the fed oil. Top vapour stream leaving the column may be partially or totally condensed by means of a top condensing unit working with an external cooling medium or, a combination of a top condensing unit working with an external cooling medium, for example, cooling water, which partially condenses the vapour stream, separating the obtained mixed vapour/liquid stream in a subsequent reflux drum, and, condensing the separated vapour stream leaving the reflux drum by using a second condensing unit working with an external cooling medium at a lower temperature than the first condensing unit, for example, chilling water. Non condensable gases leave to the vacuum unit, which can be of any available industrial type, for example, steam ejectors. Part of the condensed liquid is refluxed to the column and the rest, generates the fractionated C7 stream (40). Any water phase separated within the reflux drum is taking out as a product stream.

Styrene and ethylbenzene may be removed as a top section side product stream (30). This stream may also remove a part or the whole of xylenes and phenylacetylene, a part of the alpha-methyl styrene and other C9 aromatic compounds, a part of the acid and heteroatom containing compounds, a part of the metal containing compounds and, some of the components belonging to the fed oil fraction (C3-C8) alkanes, (C2-C8)alkenes, (C5-C8)cycloalkanes and (C5-C8)cycloalkenes with similar volatilities compared to styrene monomer.

Alpha-methyl styrene may be removed as a lower section side product stream (50). This stream may also remove a part of C9 aromatic compounds others than alpha-methyl styrene, a part of C10 aromatic compounds, a part of the acid and heteroatom containing compounds and a part of the metal containing compounds.

The heavy oif fraction may be removed as the bottom product of the column (20). This stream may also remove a part of C9 aromatic compounds and alpha-methyl styrene and a part or the whole of C10 aromatic compounds, a part of the acid and heteroatom containing compounds and a part of the metal containing compounds. This stream may also serves to provide the required latent heat for vaporization. For example, using a bottom reboiler unit which can be of any type available in the industry, for example, a forced circulating reboiler.

In accordance with another embodiment, optionally in combination with one or more features of the particular embodiments defined herein, the fractional distillation unit (T1) comprises at least one distillation column with trays, comprising from 20 to 60 theoretical stages (stage 1 - top of the column); wherein the feed of the distillation column is located at a position between 60 and 65 % of the total number of theoretical stages of the distillation column; and wherein a first fractionated pyrolysis oil sidestream (30) is obtained through a side outlet from the distillation column at a position located between 35 and 45 % of the total number of theoretical stages of the distillation column; and wherein a second fractionated pyrolysis oil sidestream (50) is obtained through a side outlet from the distillation column at a position located between 85 and 95% of the total number of theorethical stages of the distillation column.

### Option b.2) - pretreatment of the hydrocarbon liquid stream (10) using two fraction distillation units (T1+T1') - see Fig 1B

In accordance with another embodiment, optionally in combination with one or more features of the particular embodiments defined herein, step b) is carried out introducing the hydrocarbon liquid stream into a first fractional distillation unit (T1) to separate a C7 stream overhead (40) and a sidestream which is introduced into a second fractional distillation unit (T1') obtaining a first fractionated sidestream (30), a heavies containing stream (20) from bottom, and, optionally, an alpha-methyl-styrene containing product sidestream (50).

In accordance with this embodiment, the first fractional distillation unit (T1), can be a packed distillation column, a trays distillation column or a combination thereof. The column can be operated under vacuum pressure, for example 13 to 50 kPa and a temperature of 105°C in the bottom section, for example, 15 to 30 kPa, for example 20 to 30 kPa. The temperature can be greater than or equal to 80°C, for example, greater than or equal to 90°C, for example, greater than or equal to 105°C.

Top of the column consists on Benzene, Toluene, water, some of the components belonging to the fed oil fraction (C3-C8) alkanes, (C2-C8)alkenes, (C5-C8)cycloalkanes and (C5-C8)cycloalkenes with higher volatilities compared to styrene monomer, some of the acid and heteroatom containing compounds of the fed oil with higher volatities compared to styrene monomer and, part of the ethylbenzene of the fed oil. Top vapour stream leaving the column is partially or totally condensed by means of a top condensing unit working with an external cooling medium or, a combination of a top condensing unit working with an external cooling medium, for example, cooling water, which partially condenses the vapour stream, separating the obtained mixed vapour/liquid stream in a subsequent reflux drum, and, condensing the separated vapour stream leaving the reflux drum by using a second condensing unit working with an external cooling medium at a lower temperature than the first condensing unit, for example, chilling water. Non condensable gases leave to the vacuum unit, which can be of any available industrial type, for example, steam ejectors. Part of the condensed liquid is refluxed to the column and the rest generates the fractionated C7 stream (40). Any water phase separated within the reflux drum is taking out as a product stream. The balance with the total mass of oil fed to T1 is taken out of in the bottom stream of T1 and fed to T1'. This stream may also serves to provide the required latent heat for vaporization. For example, using a bottom reboiler unit which can be of any type available in the industry, for example, a forced circulating reboiler.

Whereas the second fractional distillation unit (T1') can be a packed distillation column, a trays distillation column or a combination thereof. The column can be operated under vacuum pressure, for example 13 to 65 kPa and a temperature of 180°C in the bottom section, for example, 15 to 50 kPa, for example 20 to 35 kPa. The temperature can be greater than or equal to 90°C, for example, greater than or equal to 130°C, for example, greater than or equal to 175°C.

Styrene and Ethylbenzene can be removed as a top section product stream (30). This stream may also remove a part or the whole of xylenes and phenylacetylene, a part of the alpha-methyl styrene and other C9 aromatic compounds, a part of the acid and heteroatom containing compounds, a part of the metal containing compounds and, some of the components belonging to the fed oil fraction (C3-C8) alkanes, (C2-C8)alkenes, (C5-C8)cycloalkanes and (C5-C8)cycloalkenes with similar volatilities compared to styrene monomer. Top vapour stream leaving the column is partially or totally condensed by means of a top condensing unit working with an external cooling medium or, a combination of a top condensing unit working with an external cooling medium, for example, cooling water, which partially condenses the vapour stream, separating the obtained mixed vapour/liquid stream in a subsequent reflux drum, and, condensing the separated vapour stream leaving the reflux drum by using a second condensing unit working with an external cooling medium at a lower temperature than the first condensing unit, for example, chilling water. Non condensable gases leave to the vacuum unit, which can be of any available industrial type, for example, steam ejectors. Part of the condensed liquid is refluxed to the column and the rest generates the fractionated product stream (30).

Alpha-methyl styrene can be removed as a lower section side product stream (50). This stream may also remove a part of C9 aromatic compounds others than alpha-methyl styrene, a part of C10 Aromatic compounds, a part of the acid and heteroatom containing compounds and a part of the metal containing compounds.

The Heavy oif fraction can be removed as the bottom product of the column (20). This stream may also remove a part of C9 aromatic compounds and alpha-methyl styrene and a part or the whole of C10 Aromatic compounds, a part of the acid and heteroatom containing compounds and a part of the metal containing compounds. This stream may also serves to provide the required latent heat for vaporization. For example, using a bottom reboiler unit which can be of any type available in the industry, for example, a forced circulating reboiler.

In accordance with another embodiment, optionally in combination with one or more features of the particular embodiments defined herein, the fractional distillation unit (T1) comprises at least one distillation column with trays, comprising from 10 to 30 theoretical stages (stage 1 - top of the column); wherein the feed of the distillation column is located at a position between 50 and 70 % of the total number of theoretical stages of the distillation column.

In accordance with another embodiment, optionally in combination with one or more features of the particular embodiments defined herein, the fractional distillation unit (T1') comprises at least one distillation column with trays, comprising from 15 to 40 theoretical stages (stage 1 - top of the column); wherein the feed of the distillation column is located at a position between 40 and 60 % of the total number of theoretical stages of the distillation column; and wherein a fractionated pyrolysis oil sidestream (50) is obtained through a side outlet from the distillation column at a position located between 85 and 95% of the total number of theorethical stages of the distillation column.

### Option b.3) - pretreatment of the hydrocarbon liquid stream (10) using a distillation column (D5) - see Fig 1C

In accordance with option b.3) the hydrocarbon liquid stream (10) resulting from the pyrolysis of waste plastic is directly introduced into a distillation column (D5), without being previously fractionated in any fractional distillation unit (T1 or T1'), thus obtaining a distilled out product stream (220).

The distilled out product stream (220) requires to be further hydrogenated. Therefore, distilled out product stream (220) is then fed to the hydroprocesing unit (HP) of step c.1) - see Fig 1C.

### Step c) - Hydrogenation

In the process according to the invention, step c) may be carried out according to two different alternatives, namely:
c.1) wherein the first fractionated pyrolysis oil sidestream (30) obtained in b.1) or b.2), or the distilled out product stream (220) obtained in b.3), is introduced into a hydroprocessing unit (HP) comprising an hydroprocessing catalyst in the presence of hydrogen,;
   or alternatively,
c.2) wherein the first fractionated pyrolysis oil sidestream (30) obtained in b.1) or b.2), is introduced into a distillation column (D5), the resulting distilled out product stream (220) is then fed to the hydroprocesing unit (HP) of step c.1).

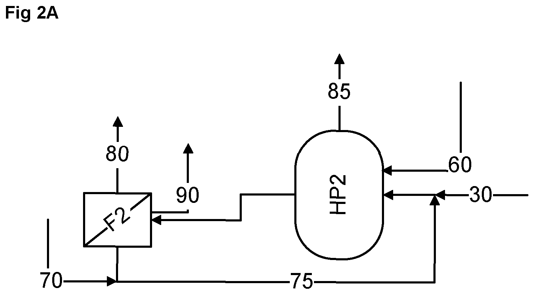

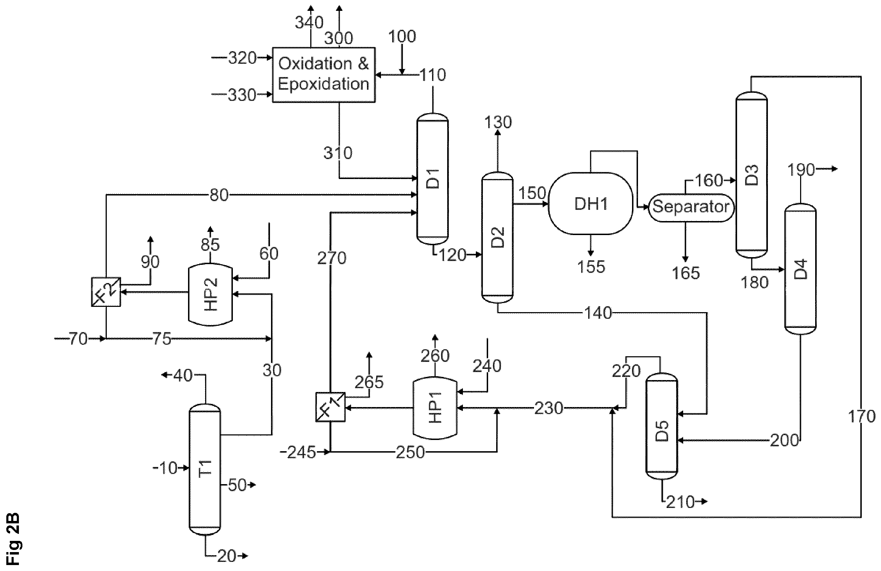

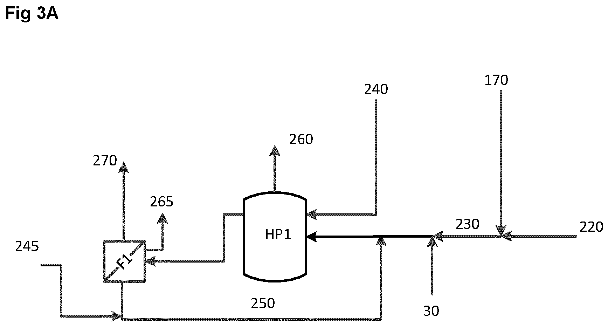

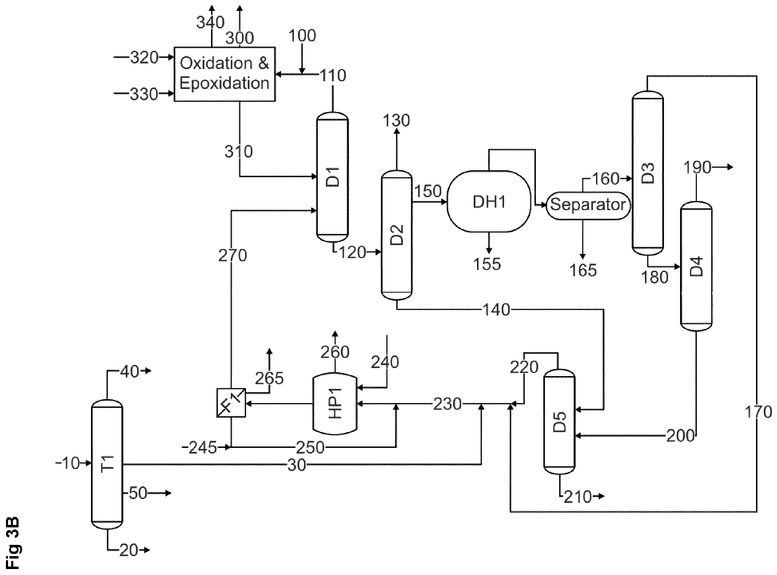

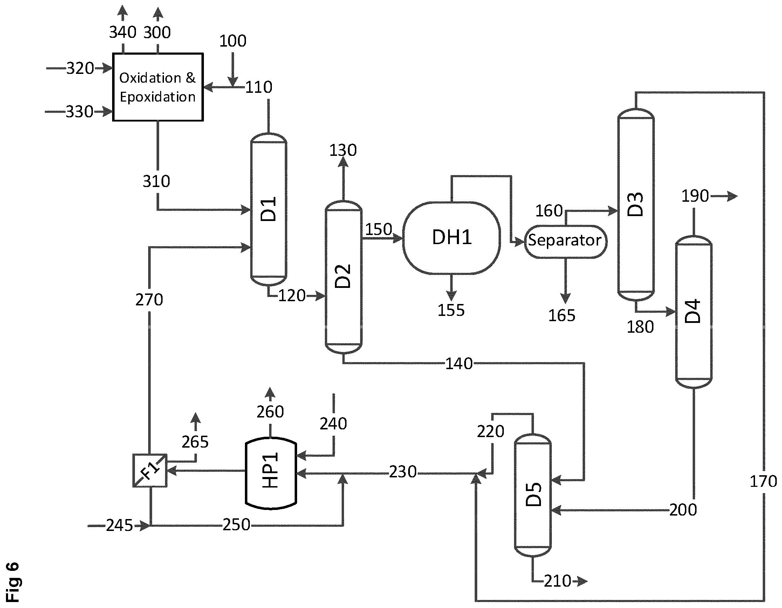

Further, depending on the hydroprocessing unit used in alternative c.1), different alternatives are contemplated:
c.1A) (see Fig 2A and 2B) the hydroprocessing unit is a dedicated hydroprocessing unit (HP2) which may comprise one or more hydrogenation reactors (HP2₁, HP2₂, ... HP2ₙ);, i.e. not the same hydroprocessing unit (HP) used for the ACP hydrogenation;
c.1B) (see Fig 3A and 3B) the hydroprocessing unit is the same hydroprocessing unit (HP1) used for the ACP hydrogenation step of the standard PO/SM process (standard PO/SM process is represented in Fig. 6), wherein the first fractionated sidestream (30) is introduced into the hydroprocessing unit (HP1) together with the distilled out product stream of the distillation column (D5).

Particular conditions may be routinely applied for the skilled person based on the technical information from common general knowledge according to the processes as already known in the art. Thus, e.g. the hydroprocessing unit (HP1) may comprise at least one slurry hydrogenation reactor or at least one fixed bed reactor. In accordance with an embodiment, this hydroprocessing unit (HP1) comprises one slurry hydrogenation reactor (HP1); or alternatively may comprise two or more slurry hydrogenation reactors (HP1₁, HP1₂... HP1ₙ).

Therefore, in the process according to the invention, step c) may be carried out according to the following alternatives:
c.1) The first fractionated sidestream (30) obtained in b.1) or b.2), or the distilled out product stream (220) obtained in b.3); is introduced into a hydroprocessing unit (HP)

This first alternative c.1), encompasses two different options:

### c.1A. Dedicated hydroprocessing unit (HP2) - see Fig 2A and 2B.

In accordance with this embodiment, the first fractionated sidestream (30) is contacted with an hydroprocessing catalyst, preferably an heterogeneous hydroprocessing catalyst, in the presence of hydrogen in at least one dedicated hydroprocessing unit (HP2), to yield a ethylbenzene containing product stream (80). The heterogeneous hydroprocesing catalyst in (HP3) may be the same heterogeneous catalyst used in the hydroprocesing unit (HP) for the ACP hydrogenation step. In accordance with one embodiment, the dedicated hydroprocessing unit (HP2) comprises one slurry hydrogenation reactor (HP2); or alternatively may comprise two or more slurry hydrogenation reactors (HP2₁, HP2₂, ... HP2ₙ) (stirred, perfect mixture, with powder catalyst) which may be working with the same catalysts of the acetophenone hydrogenation process, and which are described below.

To said hydrogenation unit is fed a catalyst (70) and hydrogen (60). From the hydrogenation unit a gaseous stream with unreacted hydrogen (85) and a slurry of hydrogenated product with catalyst is separated, which is separated from the hydrotreated liquid (80), for example by filtration (F2). Part of the separated catalyst is recovered to the hydrogenation unit (75) and part is purged (90).

The skilled person in the art knows how to select the appropriate catalyst, and the conditions to perform such a hydroprocessing step. As catalyst feed (70), it may be possible to use the "spent" catalyst from the ACP hydrogenation process (265) and, as hydrogen feed (60), the off-gas from the ACP hydrogenation unit (260). The product stream (80) can either be fed to the ACP hydrogenation unit (HP1) or mixed with the hydrotreated liquid product obtained from the ACP hydrogenation unit (270). See Fig. 2A.

### c.1B. Hydroprocessing unit (HP1) for the ACP hydrogenation step- see Fig 3A and 3B.

In accordance with this embodiment, the first fractionated sidestream (30) is contacted with an hydroprocessing catalyst, preferably a heterogeneous hydroprocessing catalyst, in the presence of hydrogen in the hydroprocessing unit (HP1) for the ACP hydrogenation step. In accordance with an embodiment, this hydrogenation unit (HP1) comprises one slurry hydrogenation reactor (HP1); or alternatively may comprise two or more slurry hydrogenation reactors (HP1₁, HP1₂... HP1ₙ) (stirred, perfect mixture, with powder catalyst) connected in series or parallel. The first fractionated sidestream (30) may be fed to one or various of such hydrogenation reactors.

Thus, in accordance with some embodiments, the hydrogenation unit (HP1) comprises two or more slurry hydrogenation reactors (HP1₁, HP1₂... HP1ₙ) connected in series with each other; wherein the first fractionated sidestream (30) is distributed between the different HP1ₙ reactors, i.e.the hydrogenation reactors are fed in parallel with the first fractionated sidestream (30); and wherein the portion of the first fractionated sidestream (30) fed to the first hydroprocessing reactor (HP1₁), yield an ethylbenzene containing product stream which is fed into the immediately subsequent reactor, and then this action is repeated for a number of n reactors until the n^{th} hydroprocessing reactor (HP1ₙ), to yield a ethylbenzene containing product stream (270). In accordance with some embodiments, the first fractionated sidestream (30) is distributed between the different HP1ₙ reactors adjusting styrene monomer concentration in the fed stream of each reactor, having lower than 15%wt styrene monomer concentration, preferably lower than 10%wt styrene monomer concentration, based on the total fed stream of each reactor.

As shown in Figures 3A and 3B, the feed introduced into to the hydroprocessing unit (HP1) comprises hydrogen (240) and a slurry containing the catalyst (250) and a mixture of the first fractionated sidestream (30), distilled out product stream (220) and ethylbenzene containing stream (170). Products out of the hydrogenation unit are a gaseous stream containing unreacted hydrogen (260) and a slurry of hydrogenated product with the catalyst, which is separated from the hydrotreated liquid (270), for example by filtration (F1). Part of the separated catalyst is recovered to the hydrogenation unit (250) and part is purged (265).

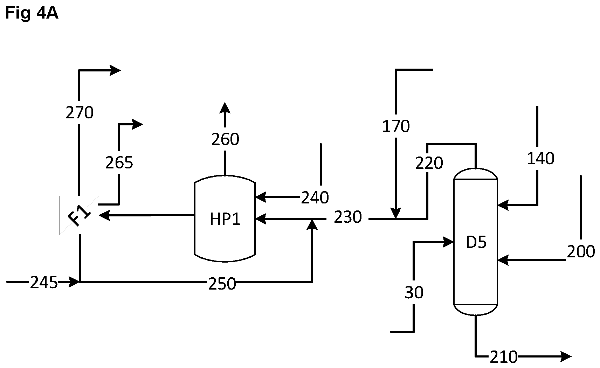

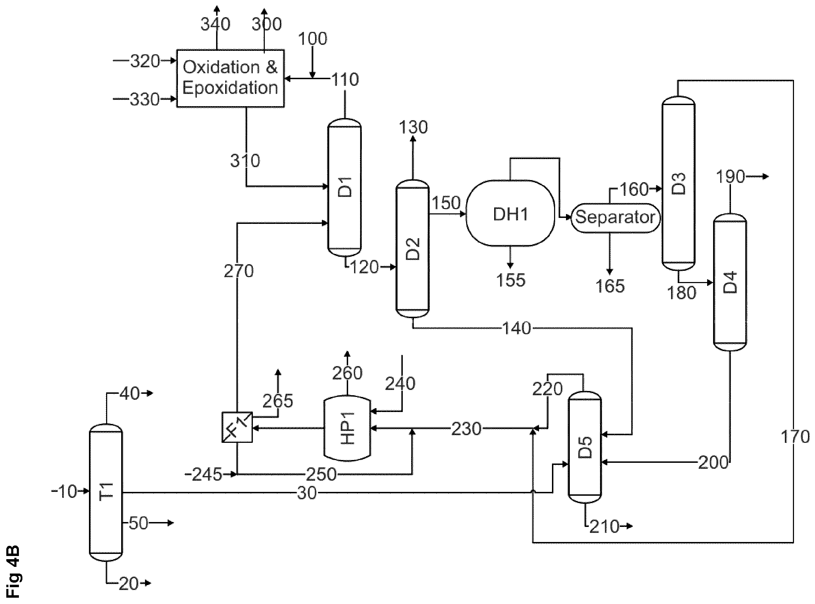

### c.2) The first fractionated sidestream (30) obtained in b.1) or b.2) is introduced into a distillation column (D5) - see Fig 4A and 4B.

In accordance with this embodiment of the present invention, the first fractionated sidestream (30) is fed to a distillation column (D5) together with the heavy bottom product streams (140) and (200), obtaining a distilled out product stream (220); the distilled out product stream (220) is fed to the hydroprocessing unit (HP) of step c.1), preferably to the hydroprocessing unit (HP1) of step c.1B), wherein it is contacted with a hydroprocessing catalyst in the presence of hydrogen to yield a ethylbenzene containing product stream (270) - see Fig 4A and 4B.

In accordance with an embodiment of this step c.1), also according to c.2), optionally in combination with one or more features of the particular embodiments defined herein, hydrogenation is carried out in a series of slurry reactors (e.g. in two or more reactors connected in serie), using e.g. a copper catalyst or a copper chromite catalyst. Particular examples of hydrogenation catalyst are well known in the art, such as Cu 0542P commertially available from BASF comprising CuO 58%wt, MnO₂ 12%wt, d50 5 microm, Scott ABD 0.4 g/ml and superfitial area 90 m²/g; or E-108P from BASF, a copper chromite catalyst comprising 35%wt Cu, 30%wt Cr, 9%wt Ba, Packed ABD 1.0 g/cc, superfitial area 40 m²/g, d50 15 microm.

In accordance with another embodiment, optionally in combination with one or more features of the particular embodiments defined herein, the process is carried out as in step c.1B), wherein at least two hydroprocessing reactors connected in series are used, and wherein the at least one portion of the first fractionated sidestream is distributed between the first hydroprocessing reactor (HP1₁) and the nth hydroprocessing reactors (HP1ₙ).

The catalyst in the different hydroprocessing reactors (HP1₁, HP1₂, ... HP1ₙ) may be same or different, as those mentioned above.

Particular conditions for operating the hydrotreatment unit (HP) may routinely applied for the skilled person based on the technical information from common general knowledge.

In accordance with a particular embodiment, optionally in combination with one or more features of the particular embodiments defined herein, in the step c), a gas purge is operated in order to maintain a hydrogen partial pressure of from 15 to 30 bara, and a temperature from 150 to 210 °C.

In accordance with an embodiment, optionally in combination with one or more features of the particular embodiments defined herein, hydrogenation of step c) is carried out in the same hydroprocessing unit (HP) used in the PO/SM process, thus step c) is carried out is carried out in the presence of an acetophenone stream, and wherein the conversion of acetophenone is between 80 to 95%, with selectivity to 1-phenyletanol of 80 to 90%, the balance ethylbenzene.

In accordance with an embodiment, optionally in combination with one or more features of the particular embodiments defined herein, in the hydrogenation step c), styrene coming into the reaction process is converted to ethylbenzene in between 95 to 100%, other C9 vinyl aromatic compounds, such as alpha-methyl-styrene are also saturated. Effluent from the last reactor is degassed, the catalyst is filtered off and recovered and, finally recycled to ethylbenzene purification (270). Catalyst purge and make-up are operated in order to maintain reaction conversion.

In accordance with a preferred embodiment, optionally in combination with one or more features of the particular embodiments defined herein, the process is carried out as in step c.2), wherein the first fractionated sidestream (30) is fed to a distillation column (D5), obtaining a distilled out product stream (220); the distilled out product stream (220) is then feed to the hydroprocesing unit (HP1) of step c.1B) wherein it is contacted with an heterogeneous hydroprocessing catalyst in the presence of hydrogen to yield the ethylbenzene product stream (270).

Particular conditions may routinely applied for the skilled person based on the technical information from common general knowledge according to the processes as already known in the art.

In accordance with another embodiment, optionally in combination with one or more features of the particular embodiments defined herein, when following either option b.1) or b.2), previously to step c), the method further comprises an alkaline or aqueous washing of the first fractionated sidestream (30). With the alkaline or aqueous washing of the first fractionated sidestream (30), it is achieved a reduction of heteroatom containing compounds present in the first fractionated sidestream (30). The presence of these heteroatom containing compounds may cause salts and acids formation, which may need to be eliminated in the process, since the may cause e.g. poisoning of catalysts.

### Step d) - Ethylbenzene recovery

In step d), the ethylbenzene containing product stream (80, 270) obtained in step c) is feed to a ethylbenzene distillation column (D1), thus a distilled ethylbenzene stream (110) is recovered from the top of the tower and a alpha-methylbenzyl alcohol containing stream (120) is recovered from bottoms. This alpha-methylbenzyl alcohol containing stream (120) also contains C8+ impurities such as xylenes, cumene, ethyltoluene, and others which come from the starting material, i.e. the hydrocarbon liquid stream resulting from the pyrolysis of waste plastic, and also from the fresh ethylbenzene stream (100).

### Step e) - Ethylbenzene oxidation

Further, in step e), and according to the well known PO/SM process (see Fig. 6), the ethylbenzene product stream (110) is incorporated, optionally together with fresh ethylbenzene (100), to a ethylbenzene oxidation unit to produce an ethylbenzene hydroperoxide stream by oxidation in liquid phase employing air, oxygen enriched air or oxygen as an oxidant (330).

Particular conditions for operating the ethylbenzene oxidation unit, wherein oxidation of ethylbenzene to ethylbenzene hydroperoxide takes place, may routinely applied for the skilled person based on the technical information from common general knowledge. For example, see Propylene Oxide PERP 2012-7 pages 19 - 20.

In accordance with an embodiment, optionally in combination with one or more features of the particular embodiments defined herein, oxidation of ethylbenzene product stream (110) to ethylbenzene hydroperoxide is carried out via uncatalyzed reaction at a temperature range of from 120 to 180°C and a pressure of from 1 to 15 bara. Adding a basic compound, for example magnesium carbonate, NaOH or KOH, to neutralize acids formed, facilitates the reaction and reduces decomposition of the hydroperoxide.

In accordance with an embodiment, optionally in combination with one or more features of the particular embodiments defined herein, oxidation step e) may be carried out to an ethylbenzene conversion ranging from 10 to 50%, preferably to about 13% conversion, in a series of stirred tank reactors. Main subproducts of the reaction are 1-phenylethanol (MBA, alpha-methylbenzyl alcohol) and acetophenone.

In accordance with an embodiment, the oxidation reactor effluent may be subjected to a separate stage where part of the ethylbenzene is removed for recycling, the reactor effluent cooled, and the ethylbenzenehydroperoxide concentration of the reactor effluent is raised.

In accordance with an embodiment, optionally in combination with one or more features of the particular embodiments defined herein, in oxidation step e) ethylbenzene feed to the process may be composed of different ethylbenzene containing streams: fresh (make-up) ethylbenzene (100), recovered ethylbenzene (110) which it is separated downstream of the epoxidation section.

Generally, commercial (fresh) ethylbenzene (100) standard specifications according to ASTM D3193-17 are as disclosed in Table 1

**Table 1. Commercial (fresh) Ethylbenzene specifications.**

| **Component** | **Specification** | **Units** |
|---|---|---|
| Ethylbenzene | 99.00 | %wt, minimum |
| Benzene | 0.1 | %wt, maximum |
| Toluene | 0.4 | %wt, maximum |
| Xylenes | 0.4 | %wt, maximum |
| Cumene | 0.03 | %wt, maximum |
| Diethylbenzene | 0.003 | %wt, maximum |
| Chlorides | 1 | mg/kg, maximum |
| Sulfur | 1 | mg/kg, maximum |

In accordance with an embodiment, optionally in combination with one or more features of the particular embodiments defined herein, the distilled ethylbenzene stream (110) comprises a ethylbenzene content from 93 to 99.5 wt%, a benzene content from 0.001 to 0.1 wt% and an aromatics C8+ (xylene, cumene, ethyltoluene, among others) content from 0.1 to 5 wt%.

In accordance with an embodiment, optionally in combination with one or more features of the particular embodiments defined herein, the distilled ethylbenzene stream (110) comprises
- from 93 to 99 wt% of ethylbenzene, preferably from 95 to 99 wt%;
- from 0.001 to 0.1 wt% of benzene, preferably from 0.01 to 0.05 wt%;
- from 0.01 to 1 wt% of toluene, preferably from 0.1 to 0.5 wt%;
- from 0.01 to 1 wt% of o-xylene, preferably from 0.1 to 0.5 wt%;
- from 0.01 to 3 wt% of m-xylene, preferably from 0.1 to 1.5 wt%;
- from 0.01 to 3 wt% of p-xylene, preferably from 0.1 to 1.5 wt%;
- from 0.01 to 1 wt% of isopropylbenzene, preferably from 0.1 to 0.5 wt%;
- from 1 to 1000 ppm wt of diethylbenzene, preferably from 10 to 100 ppm wt;
- from 0.0001 to 0.1 %wt of n-propylbenzene, preferably from 0.001 to 0.01 wt%.
- from 0.001 to 0.1 %wt of one or more compounds selected form the group consisting of a mixture of (C3-C8) alkanes, (C2-C8)alkenes, (C5-C8)cycloalkanes and (C5-C8)cycloalkenes, preferably from 0.01 to 0.06 wt%.
- from 0.01 to 50 ppmw of heteroatom containing compounds, preferably from 0.1 to 5 ppmw.
- from 0.1 to 250 ppmw of water, preferably from 0.1 to 100 ppmw.

Reaction proceeds via radicals, limiting quantities of impurities are required for feeding materials in order to preserve product selectivity.

Reaction heat may be removed by vaporization of liquid into reaction gas outlet stream. The vaporized liquid (ethylbenzene) is condensed and recycled to the reactor train. Reaction off gas non condensable products (excess oxygen used and nitrogen) stream (340) is discharged to the atmosphere after removal of trace organic (ethylbenzene and others).

In accordance with an embodiment, optionally in combination with one or more features of the particular embodiments defined herein, oxidation step e) may be carried out using air, an oxygen rich air, or pure oxygen.

### Step f) - Propylene epoxidation

The ethylbenzene hydroperoxide obtained in e) is reacted with propylene in a epoxidation unit in the presence of a catalyst to produce crude propylene oxide (300). Epoxidation step may be carried out in a fixed bed catalytic reactor equipment or in a homogeneous catalytic liquid phase reactor. Particular conditions, pressure and temperature, for these steps may be adjustable by the skilled person in the art in view of the general knowledge applicable, see for example Propylene Oxide, PERP 2012-7 page 21.

In the epoxidation of propylene to propylene oxide step using ethylbenzene hydroperoxide as epoxidating agent, particular conditions may routinely applied for the skilled person based on the technical information from common general knowledge according to the processes as already known in the art.

Thus, in accordance with a particular embodiment, the epoxidation step may takes place in the liquid phase at a temperature ranging from 80 to 150 °C, preferably 115°C and a pressure from 30 to 80 bara, preferably 40 bara, catalyzed by, e.g. a homogeneous molybdenum organometallic catalyst or a heterogeneous titania based catalyst, and employing excess propylene to improve product selectivity. Ethylbenzene hydroperoxide conversion is about 99%.

Excess propylene is distilled of the crude epoxidation product in a series of depropanizer columns operating above atmospheric pressure. Recovered propylene may be send to the reaction stage, purging build-up impurities if necessary. The bottoms product from depropanizers contains ethylbenzene (EB), 1-phenylethanol (MBA, alpha-methylbenzyl alcohol), acetophenone (ACP), propylene oxide, heavy and light reaction products. It also may contain non converted ethylbenzene hydroperoxide, acid products and molybdenum catalyst for homogeneous epoxidation process, which may be removed out of the process by liquid-liquid extraction process using aqueous alkaline solutions. Other products present like phenols, and aldehydes are also partially or totally removed in the same process.

In accordance with another embodiment, optionally in combination with one or more features of the particular embodiments defined herein, the epoxidation of propylene to propylene oxide using ethylbenzene hydroperoxide as epoxidating agent, takes place in a fixed bed equipment wherein particular conditions may be carried out according to the processes as already known in the art, e.g. it may at a temperature ranging from 50 to 130 °C and a pressure from 60 to 90 bara, over a titania-silica heterogenous catalyst in a reaction train consisting of a number of adiabatic fixed bed reactors with interstage cooling.

The skilled person in the art knows, based on the technical information from common general knowledge, how to adapt the general conditions of the epoxidation step such as propylene:ethylbenzene hydroperoxide ratio, residence time, catalyst concentration, number and disposition of reactors, as well as other reaction parameters, both for the liquid phase and the fixed bed reaction processes.

Crude propylene oxide (300) thus obtained in step f) may be introduced into a serie of propylene oxide columns that serves to separate propylene oxide from the other products. Accordingly, crude propylene oxide is refined in a serie of columns, which may be selected from normal distillation, extractive distillation or a combination of both, which removes ligther components (aldehydes, such as acetaldehyde and propionaldehyde), and heavier hydrocarbon components (benzene, toluene, propanediol, C4+ hydrocarbons) and alcohols (methanol, ethanol, 1,2-propanediol, n-propanol).

The bottoms product from the crude propylene oxide column (310) are introduced into the first ethylbenzene distillation column (D1) of step d) as described above. In the first distillation column, ethylbenzene distillation column (D1), both the bottoms product from the propylene oxide columns (310) and the ethylbenzene containing product stream (80, 270) are introduced, and a distilled ethylbenzene stream (110) is recovered from the rest of components at the top of the column. The distilled ethylbenzene stream (110) is sent upstream to the ethylbenzene oxidation unit of step e). The distilled ethylbenzene stream (110) may be optionally washed with alkali and water, in order to reduce the content of acids, alcohols, and other water soluble impurities, mainly nitrogen containing compounds.

In accordance with another embodiment, optionally in combination with one or more features of the particular embodiments defined herein, bottoms product from the crude propylene oxide column (310) comprises:
from 0.05 to 0.5 wt% of benzaldehyde;
from 0.05 to 2 wt% of mixed xylenes;
from 0.05 to 1 wt% of cumene;
from 0.001 to 0.1 wt% of n-propylbenzene;
from 0.01 to 0.5 wt% of toluene;
from 0.001 to 0.1 wt% of benzene;
from 1 to 10 wt% acetophenone;
from 25 to 40 wt% of 1-phenylethanol;
from 0.1 to 1.5 wt% of 2-phenylethanol;
from 0.1 to 5 wt% of other low vapour pressure products generated in oxidation and epoxidation reactions or introduced into the process with the first fractionated sidestream (30);
the balance to 100 wt% being ethylbezene.

### Step g) - MBA distillation

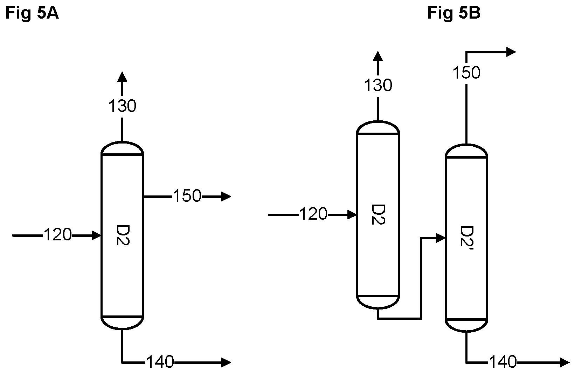

According to an embodiment of the process described herein, bottom product obtained in the ethylbenzene distillation column (D1), i.e. the alpha-methylbenzyl alcohol containing stream (120), is introduced into a second distillation column (D2), thus obtaining three fractions: a xylene containing product stream overhead (130), a alpha-methylbenzyl alcohol rich sidestream (150), and a bottom stream (140) containing alpha-methylbenzyl alcohol and higher boiling point products; the heavy bottom stream (140) is sended out to a further distillation column (D5), preferably a deep vacuum tower, in order to recover 1-phenylethanol (MBA, alpha-methylbenzyl alcohol) (210) - see Fig 5A.

Alternatively, in another embodiment, the alpha-methylbenzyl alcohol containing stream (120) is introduced into the second distillation column (D2) to separate a xylene containing stream product overhead (130), and a bottom stream which is introduced into an additional distillation column (D2'), thus obtaining a alpha-methylbenzyl alcohol rich top stream (150), and a heavy bottom stream (140) containing alpha-methylbenzyl alcohol and higher boiling point products; the heavy bottom stream (140) is sended out to a further distillation column (D5), preferably a deep vacuum tower, in order to recover 1-phenylethanol (MBA, alpha-methylbenzyl alcohol) (210) - see Fig 5B.

Xylene containing product stream (130) comprises xylenes, benzaldehide, cumene, n-propylbenzene and other aromatic C9/C10 compounds.

In accordance with an embodiment, the xylene containing product stream overhead (130) comprises mainly benzaldehyde and propanediol and, minor quantities of 1-phenylethanol, acetophenone, ethylbenzene (not separated in the previous stage), and some aromatics which may enter the process with the fresh ethylbenzene (100) and/or from the pyrolysis oil, such as: xylenes, isopropylbenzene, n-propylbenzene and diethylbenzene; the second fraction as a side product stream (150) comprises of a mixture of 75 - 85%wt 1-phenylethanol, 5 - 15%wt acetophenone and minor quantities of other components; and the bottom product (140) comprises 1-phenylethanol (MBA, alpha-methylbenzyl alcohol) and higher boiling point products.

The xylene containing product stream (130) may be purged out from the system operating at a distillate / feed mass ratio ranging from 0.001 to 0.015, preferably at a distillate / feed mass ratio of 0.005.

Particular conditions for operating the distillation columns and their technical features, may routinely applied for the skilled person based on the technical information from common general knowledge in the art.

### Step h) - MBA Dehydration

In the process of the invention, the alpha-methylbenzyl alcohol rich sidestream (150) is fed into the dehydration unit (DH1) containing a catalyst, thus obtaining crude styrene monomer (160) and water (165), which may be phase separated; also a heavy residue containing styrene oligomers (155) is obtained from bottoms of the dehydration unit (DH1)

In this step h), the catalyst used in the dehydration unit (DH1) may be an homogenoeous catalyst such as sulphuric acid, para-toluene sulfonic acid, methylsulfonic acid; or an heterogeneous catalyst such as titanium oxide, silica gel, alumina, and 1-methylimidazolium hydrogensulfate.

In accordance with a particular embodiment, dehydration reaction is a homogenously acid catalyzed dehydration reaction which takes place at a temperature ranging from 150 to 250°C, preferably 200°C and a pressure ranging from 100 to 600 mmHg, preferably 220 mmHg in the liquid phase; and wherein styrene, water, unreacted 1-phenylethanol (MBA, alpha-methylbenzyl alcohol) and acetophenone are flashed out.

In a particular embodiment, the vapors are cooled and water is phase separated.

Side reactions of the process are ethylbenzene generation and heavies generation (styrene dimer, trimer and olygomers and, 1-phenylethanol ethers). A small purge from the dehydrator liquid phase may control the builup of heavies.

In accordance with an embodiment, the method further comprises converting the heavy residue containing styrene oligomers (155), which is obtained in the dehydration unit (DH1), into a pyrolysis unit, to a recycled hycrocarbon liquid stream (recycled pyrolysis oil stream) (10'), which may be further introduced into the frist fractional distillation unit (T1) or to the distillation column (D5).

### Step i) - Styrene purification

The styrene purification section comprises a sequence of two distillation columns (D3 and D4). The first column (D3) is used to eliminate lights (170) from crude styrene, which are sent to the hydroprocessing unit (HP). The styrene product is obtanined from the top of the second column (D4), whereas bottoms (200) from this second column (D4) is sent to the distillation column (D5).

Thus, according to an embodiment, the crude styrene (160) is fed to a distillation column (D3) where ethylbenzene containing stream (170), comprising ethylbenzene (preferably from 1 to 10 wt%, more preferably from 2 to 6 wt%, more preferably about 4 wt% in styrene) is top separated in order to cope with the styrene product quality specification. This ethylbenzene containing stream (170) is sent to the hydroprocessing unit (HP). Bottom product (180) obtained from (D3) is fed to the styrene product column (D4) where pure (>99,8 wt%) styrene monomer is obtained at the top (190) and separated from non converted 1-phenylethanol (MBA, alpha-methylbenzyl alcohol) and acetophenone (ACP) which are obtained as a bottom product (200). This MBA/ACP containing bottom product (200) is sent to the distillation column (D5).

### Step j) - Acetophenone recovery

In accordance with an embodiment, optionally in combination with one or more features of the particular embodiments defined herein, "heavy bottom" product streams (140) and (200) are fed to the distillation unit (D5).

In accordance with an embodiment, the distillation unit (D5) may comprise a distillation column (D5) or a serie of distillation columns (D5n) so to deep recover valuable components carried over in those streams from the previous separation operations: acetophenone, 1-phenylethanol, and styrene. Distilled out product stream (220) is fed alongside styrene/ethylbenzene stream (170) to the hydroprocesing unit (HP).

See Fig 6 for a general schematic description of the PO/SM process.

The distillation column (D5) may be same or different from the distillation column (D5) of steps b3) and c.2), and the hydroprocesing unit (HP) may be same or different to the hydroprocesin unit (HP) of step d).

Thus, in accordance with some embodiments, the distillation column (D5) of step j) is the same distillation column of b.3) wherein the hydrocarbon liquid stream (10) resulting from the pyrolysis of waste plastic is distilled together with the heavy bottom product streams (140) and (200) to obtain a distilled out product stream (220) which is then fed to the hydroprocesing unit (HP) of step c.1). See Fig. 1C.

In accordance with another embodiment, the distillation column (D5) of step j) is the same distillation column of c.2) wherein the first fractionated sidestream (30) obtained according to b.1) or b.2) is distilled together with the heavy bottom product streams (140) and (200) to obtain a distilled out product stream (220) which is then fed to the hydroprocesing unit (HP) of step c.1). See Figs. 4A and 4B.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim and shall not be construed as limiting the scope of the claim. Furthermore, the present invention covers all possible combinations of particular and preferred embodiments described herein.

### Examples

### 1. Hydrocarbon liquid stream

In the following examples, the hydrocarbon liquid stream (pyrolysis oil 1) is an oil obtained by pyrolysis of polystyrene having at least 50% of SM (see US10301235, WO2021230312 and WO2021053074 having a composition typo as disclosed below, and which is not further purified). A composition typo of pyrolysis oil 1 is as desclosed in table 2.

**Table 2. Composition of the polystyrene pyrolysis oil**

| **Components** | **polystyrene pyrolysis oil content wt%** |
|---|---|
| Styrene monomer (SM) | 65.2 |
| Ethylbenzene (EB) | 2.7 |
| phenylacetylene | 0.1 |
| Cumene | 0.3 |
| Alfa methyl styrene (AMS) | 5.5 |
| Toluene | 7.0 |
| benzene | 2.0 |
| C8 aromatic compounds^{(a)} | 1.0 |
| C9 aromatic compounds^{(b)} | 1.9 |
| C₃-C₈ fraction ^{(c)} | 0.7 |
| C10 aromatic compounds^{(d)} | 0.8 |
| heavy oil fraction^{(e)} | 12.19 |
| heteroatom containing compounds | 0.41 |
| Water | 0.2 |

| | |
|---|---|
| (a) a mixture comprising; ortho-xylene, meta-xylene, and para-xylene, among others; (b) a mixture comprising propenylbenzene, n-propylbenzene; allyl-benzene, ethyl-toluene and methyl-styrene, among others; (c) C3-C8 fraction refers to the fraction comprising one or more compounds selected form the group consisting of (C3-C8)alkanes, (C3-C8)alkenes, (C5-C8)cycloalkanes and (C5-C8)cycloalkenes; (d) a mixture comprising of divinylbenzene and ethyl-styrene; (e) a mixture comprising naphthalene, bi-benzyl, dimers, trimers , and stilbene among others. | |

See US10301235, WO2021230312 and WO2021053074 for processes of preparation of hydrocarbon liquid streams by pyrolysis of waste plastics having a composition typo as disclosed above.

### 2. Fractional distillation unit

Two different configurations of fractional distillation were used:
- Option (b.1): introducing of the hydrocarbon liquid stream into a first fractional distillation unit (T1) to separate a C7 stream overhead (40), a first fractionated sidestream (30), a heavies-containing stream (20) from bottom, and, optionally, an alpha-methyl-styrene containing product sidestream (50); see figure 1A; and
- Option (b.2): the hydrocarbon liquid stream is introduced into a first fractional distillation unit (T1) to separate a C7 stream overhead (40) and a sidestream which is introduced into a second fractional distillation unit (T1') obtaining a first fractionated sidestream (30), a heavies containing stream (20) from bottom, and, optionally, an alpha-methyl-styrene containing product sidestream (50); see figure 1B.

The resulting first fractionated sidestream (30) thus obtained comprise the compositions disclosed in Table 3.

**Table 3. First fractionated sidestream (30)**

| **Components** | **first fractionated sidestream (30) method option b.1)** | **first fractionated sidestream (30) method option b.2)** |
|---|---|---|
| Styrene monomer (SM) wt% | 90.8376 | 90.7252 |
| Ethylbenzene (EB) wt% | 3.3813 | 3.7338 |
| Phenylacetylene wt% | 0.1335 | 0.1390 |
| Cumene wt% | 0.4179 | 0.4099 |
| Alfa methyl styrene (AMS) wt% | 2.4736 | 2.4997 |
| Toluene wt% | 0.3097 | 0.0628 |
| Benzene wt% | 0.0250 | 0.0000 |
| C8 aromatic compounds^{(a)} wt% | 1.3887 | 1.3859 |
| C9 aromatic compounds^{(b)} wt% | 0.7520 | 0.7545 |
| C₃-C₈ fraction ^{(c)} wt% | 0.6933 | 0.6964 |
| C10 aromatic compounds^{(d)} wt% | 0.0000 | 0.0001 |
| Heavy oil fraction^{(e)} wt% | 0.0053 | 0.0025 |
| heteroatom containing compounds ppmw | | |
| Water ppmw | 105 | 0 |
| 30/10 mass ratio | 0.716 | 0.716 |

| | | |
|---|---|---|
| (a) a mixture comprising ortho-xylene, meta-xylene, and para-xylene, among others; (b) a mixture comprising propenylbenzene, n-propylbenzene; allyl-benzene, ethyl-toluene and methyl-styrene, among others; (c) C3-C8 fraction refers to the fraction comprising one or more compounds selected form the group consisting of (C3-C8)alkanes, (C3-C8)alkenes, (C5-C8)cycloalkanes and (C5-C8)cycloalkenes; (d) a mixture comprising of divinylbenzene and ethyl-styrene; (e) a mixture comprising naphthalene, bi-benzyl, dimers, trimers , and stilbene among others. | | |

Following method (b.1), a pyrolysis oil (10) as disclosed above was introduced into a distillation column with a reboiler at the bottom and a partial condenser (liquid / vapor) at the head. The column worked under vacuum. The head condenser run with cooling water up to a vapor temperature of 50°C. The noncondensed vapor passed to a refrigeration chiller where it was partially condensed at reduced temperatures with cooling fluid (e.g. subcooled glycol water solution). The condensed hydrocarbon joined with the column distillate generating the C7- cut overhead (40).

Thus, the column works with partial reflux, separating by rectification in the head section of the column the benzene, toluene and light hydrocarbons (40) from the aromatic C8 side cut (30) rich in SM and EB. The pyrolysis oil (10) was fed below the side extraction of the C8 side cut (30).

In the bottom section of the column, a stream rich in AMS (50) was obtained as a side liquid extraction.

At the bottom of the column, the vapors for distillation were generated by means of a reboiler, which can be of the types known in the state of the art, and the heavy stream, cut C9+ (20), was separated.

The example was obtained by computer simulation employing UNIFAC properties method (using Aspen Plus modeling Software). Below are the process conditions used:
- 30 Theoretical stages. Feed in stage 19 (above) and lateral extraction of C8 cut in stage 12 and, AMS cut in stage 27 (stage 1 = condenser; stage 30 = boiler or reboiler).
- Reflux Ratio: 116
- C8 cut to feed mass ratio: 0.716
- AMS cut to feed mass ratio: 0.045
- Bottoms to feed mass ratio: 0.137
- Condenser: partial-vapor-liquid. Temperature 50°C.
- Condenser vapor Chiller Temperature: 5°C
- Pressure profile: stage 1 - 155 mmHg; stage 2 - 175 mmHg; Pressure Drop rest of the column - 20 mmHg.

Process simulation in said above conditions provides the following results (Table 4) in conditions and composition on key components for the main pyrolysis oil fractionation streams.

**Table 4. Polystyrene pyrolysis oil fractionation results (%wt) (Method b.1)**

| | (10) PYOIL | (20) C9⁺ | (30) SM | (40) C7⁻ | (50) AMS |
|---|---|---|---|---|---|
| Styrene monomer (SM) | 65.2314 | 0.0053 | 90.8376 | 2.3288 | 0.3186 |
| Ethylbenzene (EB) | 2.7001 | 0.0000 | 3.3813 | 2.8137 | 0.0005 |
| phenylacetylene | 0.1000 | 0.0000 | 0.1335 | 0.0450 | 0.0000 |
| Cumene | 0.3000 | 0.0005 | 0.4179 | 0.0013 | 0.0191 |
| Alfa methyl styrene (AMS) | 5.5001 | 5.0161 | 2.4736 | 0.0000 | 67.6833 |
| Toluene | 7.0001 | 0.0000 | 0.3097 | 67.4045 | 0.0000 |
| benzene | 2.0000 | 0.0000 | 0.0250 | 19.1160 | 0.0000 |
| C8 aromatic compounds^{(a)} | 1.0000 | 0.0002 | 1.3887 | 0.0582 | 0.0147 |
| C9 aromatic compounds^{(b)} | 1.9000 | 2.9338 | 0.7520 | 0.0013 | 21.3573 |
| C3-C8 fraction ^{(c)} | 0.7000 | 0.0000 | 0.6933 | 1.2833 | 0.0017 |
| C10 aromatic compounds^{(d)} | 0.8000 | 4.2785 | 0.0000 | 0.0000 | 4.7727 |
| heavy oil fraction^{(e)} | 13.0683 | 87.7661 | 0.0053 | 6.9492 | 5.8512 |
| Water | 0.2000 | 0.0000 | 0.0001 | 1.2994 | 0.0000 |
| Product to feed mass ratio | - | 0.137 | 0.716 | 0.100 | 0.045 |
| Temperature (°C) | 25.0 | 178.0 | 98.1 | 18.1 | 121.6 |
| Pressure (kg/cm²) | 2 | 0.265 | 0.248 | 0.208 | 0.262 |

| | | | | | |
|---|---|---|---|---|---|
| (a) a mixture comprising ortho-xylene, meta-xylene, and para-xylene, among others; (b) a mixture comprising propenylbenzene, n-propylbenzene; allyl-benzene, ethyl-toluene and methyl-styrene, among others; (c) C3-C8 fraction refers to the fraction comprising one or more compounds selected form the group consisting of (C3-C8)alkanes, (C3-C8)alkenes, (C5-C8)cycloalkanes and (C5-C8)cycloalkenes; (d) a mixture comprising of divinylbenzene and ethyl-styrene; (e) a mixture comprising naphthalene, bi-benzyl, dimers, trimers , and stilbene among others. | | | | | |

In the SM product (30) it is observed that the C8's and cumene were not separated but, the Benzene, Toluene and, the heavy ones together with a part of the AMS and N-propylbenzene were eliminated, with a SM recovery of 99% (and 89% of the EB), obtaining a rich stream in SM+EB (approx. 94%wt and, 2.5%wt maximum of AMS)

Following method (b.2), a pyrolysis oil (10) as disclosed above was introduced into a first distillation column with a reboiler at the bottom and a partial condenser (liquid / vapor) at the head. The column worked under vacuum. The head condenser run with cooling water up to a vapor temperature of 40°C. The noncondensed vapor passed to a refrigeration chiller where it was partially condensed at reduced temperatures with cooling fluid (e.g. subcooled glycol water). The condensed hydrocarbon joined with the column distillate generating the C7- cut overhead (40).

Thus, the column works with partial reflux, separating by rectification in the head section of the column the benzene, toluene and light hydrocarbons (40).

At the bottom of the column, the vapors for distillation were generated by means of a reboiler, which can be of the types known in the state of the art, and the bottom stream so obtained, is fed to a second distillation column with a reboiler at the bottom and a total condenser at the head. The column worked under vacuum.

Thus, the column works with partial reflux, separating by rectification a top distillate stream rich in SM (30).

In the bottom section of the column, a stream rich in AMS (50) was obtained by lateral liquid extraction.

At the bottom of the column, the vapors for distillation were generated by means of a reboiler, which can be of the types known in the state of the art, and the heavy stream, cut C9+ (20), was separated.

The example was obtained by computer simulation employing UNIFAC properties method (using Aspen Plus modeling Software). Below are the process conditions used:
First distillation column:
   - 15 Theoretical stages. Feed in stage 8 (above) (stage 1 = condenser; stage 15 = boiler or reboiler).
   - Reflux Ratio: 19.5
   - Distillate to feed mass ratio: 0.1
   - Condenser: partial-vapor-liquid. Temperature 40°C.
   - Condenser vapor Chiller Temperature: 5°C
   - Pressure profile: stage 1 - 175 mmHg; stage 2 - 180 mmHg; Pressure Drop rest of the column - 30 mmHg.
Second distillation column:
   - 20 Theoretical stages. Feed in stage 10 (above) and lateral extraction of AMS cut in stage 18 (stage 1 = condenser; stage 20 = boiler or reboiler).
   - Reflux Ratio: 0.985
   - AMS cut to feed mass ratio: 0.045
   - Distillate to feed mass ratio: 0.795
   - Condenser: total
   - Pressure profile: stage 1 - 155 mmHg; stage 2 - 160 mmHg; Pressure Drop rest of the column - 30 mmHg.

Process simulation in said above conditions provides the following results (Table 5) in conditions and composition on key components for the main pyrolysis oil fractionation streams.

**Table 5. Polystyrene pyrolysis oil fractionation results (%wt) (Method b.2).**

| | (10) PYOIL | (20) C9⁺ | (30) SM | (40) C7⁻ | (50) AMS |
|---|---|---|---|---|---|
| Styrene monomer (SM) | 65.2314 | 0.1499 | 90.7252 | 0.1680 | 4.0204 |
| Ethylbenzene (EB) | 2.7001 | 0.0003 | 3.7338 | 0.2562 | 0.0128 |
| phenylacetylene | 0.1000 | 0.0000 | 0.1390 | 0.0043 | 0.0009 |
| Cumene | 0.3000 | 0.0046 | 0.4099 | 0.0001 | 0.1028 |
| Alfa methyl styrene (AMS) | 5.5001 | 5.0001 | 2.4997 | 0.0000 | 53.6503 |
| Toluene | 7.0001 | 0.0000 | 0.0628 | 69.5812 | 0.0000 |
| benzene | 2.0000 | 0.0000 | 0.0000 | 20.0089 | 0.0000 |
| C8 aromatic compounds^{(a)} | 1.0000 | 0.0041 | 1.3859 | 0.0075 | 0.1116 |
| C9 aromatic compounds^{(b)} | 1.9000 | 2.4558 | 0.7545 | 0.0002 | 18.2862 |
| C3-C8 fraction ^{(c)} | 0.6000 | 0.0005 | 0.6964 | 1.0031 | 0.0172 |
| C10 aromatic compounds^{(d)} | 0.8000 | 3.1716 | 0.0001 | 0.0000 | 6.9454 |
| heavy oil fraction^{(e)} | 13.1682 | 89.2177 | 0.0025 | 8.9707 | 16.9552 |
| Water | 0.2000 | 0.0000 | 0.0000 | 2.0009 | 0.0000 |
| Product to feed mass ratio | - | 0.127 | 0.716 | 0.100 | 0.057 |
| T (°C) | 25.0 | 177.7 | 92.9 | 21.0 | 120.6 |
| P (kg/cm²) | 2.0 | 0.245 | 0.204 | 0.235 | 0.240 |

| | | | | | |
|---|---|---|---|---|---|
| (a) a mixture comprising ortho-xylene, meta-xylene, and para-xylene, among others; (b) a mixture comprising propenylbenzene, n-propylbenzene; allyl-benzene, ethyl-toluene and methyl-styrene, among others; (c) C3-C8 fraction refers to the fraction comprising one or more compounds selected form the group consisting of (C3-C8)alkanes, (C3-C8)alkenes, (C5-C8)cycloalkanes and (C5-C8)cycloalkenes; (d) a mixture comprising of divinylbenzene and ethyl-styrene; (e) a mixture comprising naphthalene, bi-benzyl, dimers, trimers , and stilbene among others. | | | | | |

For heteroatom containing compounds process simulation is not feasible. A batch distillation experimental test employing a 15 theoretical stages distillation column operating with a reflux ratio of 15 and 100 mmHg of pressure has been perfomed with the results disclosed in Table 6.

**Table 6. Polystyrene pyrolysis oil fractionation. Heteroatom distribution (t)**

| | (40) C7⁻ | (30) SM | (20) C9⁺ |
|---|---|---|---|
| Temperature (@760 mmhg) (°C) | < 140 | 140 - 150 | >150 |
| Product to feed mass ratio | 0.038 | 0.81 | 0.152 |
| Acidity product to feed mass ratio | 0.526 | 0.101 | 0.373 |
| Chlorine product to feed mass ratio | 0.142 | 0.31 | 0.547 |
| Sulfur product to feed mass ratio | 0.151 | 0.532 | 0.316 |
| Nitrogen product to feed mass ratio | 0.357 | 0.28 | 0.364 |
| Bromide product to feed mass ratio | 0.24 | 0.425 | 0.335 |
| Fluoride product to feed mass ratio | 0.59 | 0.209 | 0.732 |

### 3. Effect of incorporation of the hydrocarbon liquid stream (10) into the POSM process

### Comparative example 1. POSM process without incorporation of (10)

A process flow diagram of the Propylene Oxide / Styrene Monomer (PO/SM) process for producing styrene via ethylbenzene hydroperoxide intermediate is shown in Figure 6. These flow diagram do not show the ethylbenzene oxidation and ethylbenzene hydroperoxide epoxidation sections of the process, neither the propylene oxide purification part of the process.

The first step in the process was the air oxidation of ethylbenzene to ethylbenzene hydroperoxide via uncatalyzed reaction at approximately 150°C and 2.8 bara. This reaction was carried out to about 13% ethylbenzene conversion in a series of stirred tank reactors. Selectivity to ethylbenzene hydroperoxide was 80%. Main subproducts of the reaction are 1-phenylethanol (MBA, alpha-methylbenzyl alcohol) and acetophenone (ACP). Minor subproducts are aldehydes, acids and phenols, obtained from secondary oxidation reactions. Ethylbenzene feed to the process is composed of two streams, fresh (make-up) ethylbenzene (100) and recovered Ethylbenzene (110) which it was separated downstream of the epoxidation section.

Table 7 shows the considered Ethylbenzene make-up stream (which will be the same for all the examples herein disclosed):

**Table 7. Make-up Ethylbenzene (100).**

| **Compound** | **Composition (%wt)** |
|---|---|
| Ethylbenzene | 99.9121 |
| Benzene | 0.0619 |
| Toluene | 0.0099 |
| C8 aromatic compounds^{a} | 0.0054 |
| Cumene | 0.0046 |
| C9 aromatic compounds^{b} | 0.0038 |
| C10 aromatic compounds^{c} | 0.0023 |

| | |
|---|---|
| (a) a mixture comprising; ortho-xylene, meta-xylene, and para-xylene; (b) mainly n-propylbenzene; (c) mainly diethylbenzene; | |

Reaction heat was removed by vaporization of liquid into reaction gas outlet stream. The vaporized liquid (ethylbenzene) was condensed and off-gas (excess oxygen used and nitrogen) was discharged to the atmosphere after removal of trace organic (ethylbenzene and others).

The next step in the process is the epoxidation of propylene to propylene oxide using ethylbenzene hydroperoxide concentrated up to 35% wt, as epoxidating agent, which also yields 1-phenylethanol. Reaction was carried out at 115°C and 40 bara in the liquid phase, catalyzed by a molybdenum organometallic catalyst and employing excess propylene to improve product selectivity. Ethylbenzene hydroperoxide conversion was about 99%. Excess propylene was distilled of the crude epoxidation product in a series of depropanizer columns operating above atmospheric pressure. Recovered propylene was send to the reaction stage, purging build-up impurities if necessary and adding fresh propylene (320). The bottoms product from depropanizers cotains ethylbenzene (EB), 1-phenylethanol (MBA, alpha-methylbenzyl alcohol), acetophenone (ACP), propylene oxide, heavy and light reaction products. It also contains non converted ethylbenzene hydroperoxide, acid products and molybdenum catalyst, which were removed out of the process by liquid-liquid extraction process using aqueous alkaline solutions. Other products present like phenols, and aldehydes were also partially or totally removed in the same process.

Latter product stream was sent to a crude propylene oxide column that serves to separate crude propylene oxide (300) from the other products. The crude propylene oxide was refined in a series of columns which removes ligther components (aldehydes, such as acetaldehyde and propionaldehyde), and heavier hydrocarbon components (benzene, toluene, propanediol, C4+ hydrocarbons) and alcohols (methanol, ethanol, 1,2-propanediol, n-propanol).

The bottoms product from the crude propylene oxide column was sent to a sequence of two columns (D1 and D2). In the first distillation column (D1), ethylbenzene was recovered from the rest of components at the top of the column (110) and, sent upstream to the ethylbenzene oxidation section. The bottom product was fed to the second distillation tower (D2) where three fractions were obtained: a top stream product (130) which contains mainly benzaldehyde and propanediol and, minor quantities of 1-phenylethanol, acetophenone, ethylbenzene (not separated in the previous stage), and some aromatics which may enter the process with the fresh ethylbenzene (100) such as: xylenes, isopropylbenzene, n-propylbenzene and diethylbenzene; a second fraction as a side product stream (150) composed of a mixture of 80%wt 1-phenylethanol, 10%wt acetophenone and minor quantities of other components; and, finally, the bottom product (140) which contains some 1-phenylethanol (MBA, alpha-methylbenzyl alcohol) and higher boiling point products, which is sent out to a deep vacuum tower (D5) in order to recover 1-phenylethanol.

**Table 8. Oxidaton and epoxidation by-products generation. Comparative example 1.**

| **Product** | **t / t SM product*** |
|---|---|
| Benzaldehyde | 0.0020 |
| Acetophenone | 0.1617 |
| 1-phenylethanol | 1.0968 |
| Benzyl-alcohol | 0.0152 |
| 2-phenylethanol | 0.0127 |
| 1-phenyl-2-propanol | 0.0254 |
| 1,1'-oxybis(ethane-1,1-diyl)dibenzene & isomers | 0.0254 |
| oxidation & epoxidation heavies | 0.0017 |

| | |
|---|---|
| * "t/t SM product" means metric tonnes of product per metric tonnes of styrene monomer stream (190) | |

The 1-phenylethanol (MBA, alpha-methylbenzyl alcohol) / acetophenone (ACP) stream (150) was fed into the dehydration reactor. The homogenously acid catalyzed dehydration reaction was carried out at 200°C and 220 mmHg in the liquid phase and styrene, water, unreacted 1-phenylethanol (MBA, alpha-methylbenzyl alcohol) and acetophenone were flashed out. The vapors were cooled and water was phase separated. Side reactions of the process were ethylbenzene generation and heavies generation (styrene dimer, trimer and olygomers and, 1-phenylethanol ethers). A small purge from the dehydrator liquid phase controls the builup of heavies.

The crude styrene (160) was sent to a lights column (D3) where some ethylbenzene (about 4%wt in Styrene) was top separated (170) in order to cope with the styrene product quality specification. Bottom product was fed to the styrene product column (D4) where pure (>99,8 %wt) styrene monomer was obtained at the top (190) and separated from non converted 1-phenylethanol (MBA, alpha-methylbenzyl alcohol) and acetophenone which were obtained as a bottom product (200).

"Heavy bottom" product streams (140) and (200) off the process were fed to a deep vacuum tower (D5) or series of distillation towers so to deep recover valuable components carried over in those streams from the previous separation operations: 1-phenylethanol, acetophenone and styrene. Distilled out product stream (220) was fed alongside styrene/ethylbenzene stream (170) to the hydrogenation reaction section. Hydrogenation was carried out experimentally in a one liter autoclave reactor using a copper oxide-type catalyst. 500 grams of a liquid with the composition given in the table 9 and 1.5 g of the catalyst Cu 0542 P from BASF were charged in the reactor. Reactor was closed, purged with nitrogen and purged with pure hydrogen. Then, reactor content was heated up under stirring to 188°C. Pure hydrogen (>99.99%vol) was fed maintaining a total pressure of 24 bara and controlling temperature at 188°C over a period of 180 minutes. Afterwards, reactor was cooled down, pressure was reduced up to atmospheric and, product discharged to analysis. Table 9 shows main results obtained.

**Table 9.**

| **Feed Composition** | |
|---|---|
| Ethylbenzene (%wt) | 0.005 |
| Styrene (%wt) | 0.245 |
| Acetophenone (%wt) | 43.115 |
| 1-phenyletanol (%wt) | 20.55 |
| Alpha methyl styrene (%wt) | 0 |
| Reaction Results | |
| Styrene conversion % | 98.9 |
| Acetophenone conversión % | 94.3 |
| 1-phenylethanol to acetophenone Selectivity % | 83.5 |

In another example, hydrogenation was carried out in a series of two slurry reactors using a 0.2%wt copper chromite-type catalyst. Hydrogen (90%vol) was fed to each reactor and, a gas purge was operated in order to maintain the required hydrogen partial pressure at about 24 bara. Temperature was maintained at about 190°C. Total residence time was about 2,5 hours. Table 10 shows main results obtained.

**Table 10.**

| **Feed Composition** | |
|---|---|
| Ethylbenzene (%wt) | 2.03 |
| Styrene (%wt) | 1.53 |
| Acetophenone (%wt) | 51.65 |
| 1-phenyletanol (%wt) | 28.78 |
| Alpha methyl styrene (%wt) | 0 |
| Reaction Results | |
| Styrene conversion % | 98.5 |
| Acetophenone conversión % | 88.3 |
| 1-phenylethanol to acetophenone Selectivity % | 90.7 |

### Comparative example 2. PO/SM process with incorporation of fractionated styrene product stream (30) to crude styrene distillation column (D3)

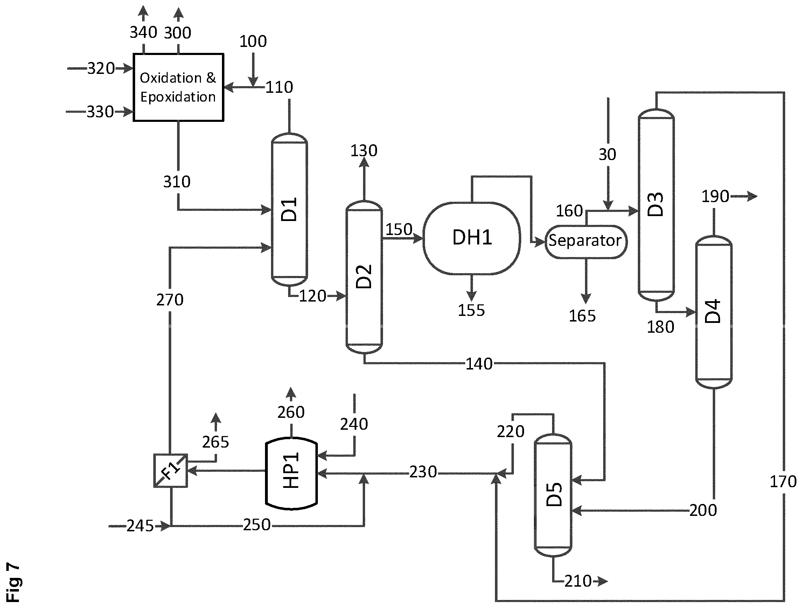

0.026 tonnes of polystyrene pyrolysis oil per ton of styrene monomer production of the comparative example 1 process were fractionated as previously described above (method b1). Fractionated styrene product stream (30) was fed to crude styrene distillation (D3) mixed with crude styrene product of dehydrator phase separator (see Figure 7). Rest of the process is same as comparative example 1.

### Comparative example 3. PO/SM process with incorporation of polystyrene pyrolysis oil (10) to crude styrene distillation column (D3)

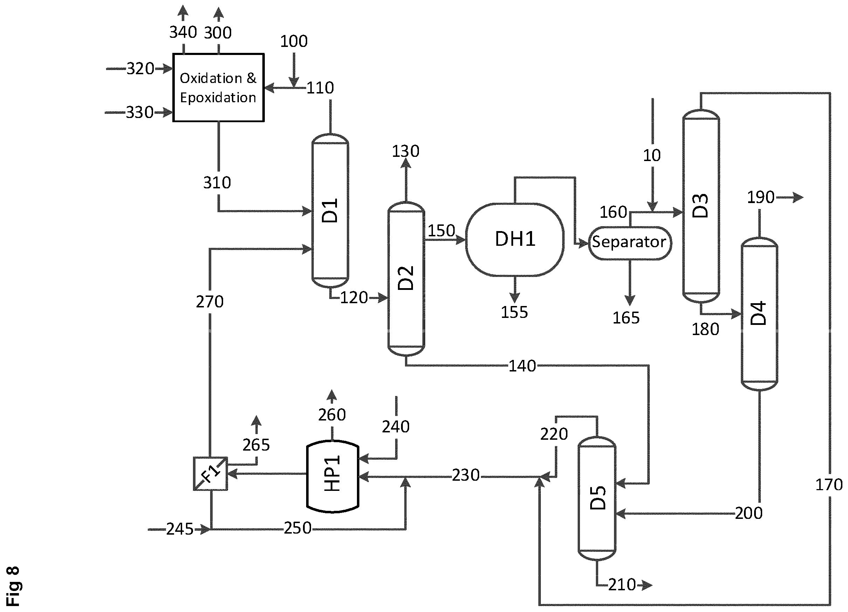

0.026 tonnes of polystyrene pyrolysis oil per ton of styrene monomer production of the comparative example 1 were fed to crude styrene distillation (D3) mixed with crude styrene product of dehydrator phase separator (see Figure 8). Rest of the process is same as comparative example 1.

### Example 1 according to the invention. PO/SM process with incorporation of fractionated styrene product stream (30) to the hydrogenation unit (HP) - Fig 3B

0.026 tonnes of polystyrene pyrolysis oil per ton of styrene monomer production of the comparative example 1 were fractionated as previously described above (method option b.1). Fractionated styrene product stream (30) was fed to hydrogenation unit (HP) as described in method c.1B. Rest of the process is same as comparative example 1.

Hydrogenation was carried out in a one liter autoclave reactor using a copper oxide-type catalyst. 426 grams of a rich acetophenone liquid with a composition like comparative example 1; 6.8 grams of a 96 %wt styrene, 4%wt ethylbenzene mixture and, 16.7 grams of fractionated polystyrene pyrolysis oil as previously described were mixed giving the composition given in the table 9 and 1.35 g of the catalyst Cu 0542 P from BASF were charged in the reactor. Reactor was closed, purged with nitrogen and purged with pure hydrogen. Then, reactor content was heated up under stirring to 188°C. Pure hydrogen (>99.99%vol) was fed maintaining a total pressure of 24 bara and controlling temperature at 188°C over a period of 180 minutes. Afterwards, reactor was cooled down, pressure was reduced up to atmospheric and, product discharged to analysis. Used catalyst was filtered off and recovered for a second used following same procedure just adding 68 milligrams of fresh catalyst. Table 11 shows main results obtained.

**Table 11.**

| **Feed composition** | |
|---|---|
| Ethylbenzene %wt | 0.497 |
| Styrene %wt | 3.806 |
| Acetophenone %wt | 43.226 |
| 1-phenyletanol %wt | 18.139 |
| Alpha methyl styrene %wt | 0.284 |
| Chloride ppmw | 4.7 |
| Bromide ppmw | 0.5 |
| Fluoride ppmw | 0.3 |
| Nitrogen ppmw | 150 |
| Sulfur ppmw | 0.3 |
| Acidity, mgKOH/g | 0.008 |
| Results obtained 1st catalyst use | |
| Styrene conversion % | 97.48 |
| Acetophenone conversión % | 95 |
| Alpha methyl styrene conversion | 98.9 |
| 1-phenylethanol to acetophenone Selectivity % | 83 |
| Results obtained 2nd catalyst use | |
| Styrene conversion % | 96.5 |
| Acetophenone conversion % | 95 |
| Alpha methyl styrene conversion | 83.1 |
| 1-phenylethanol to acetophenone Selectivity % | 70 |

### Example 2 according to the invention. PO/SM process with incorporation of fractionated styrene product stream (30) to the distillation unit (D5) - Fig 4B

0.026 tonnes of polystyrene pyrolysis oil per ton of styrene monomer production of the comparative example 1 were fractionated as previously described above (method option b.1). Fractionated styrene product stream (30) was fed to distillation unit (D5) as described in method c.2). Rest of the process is same as comparative example 1.

Key results for all examples described above are depicted in the following tables:

**Table 12. Styrene monomer stream (190)**

| | **Ex 1** | **Comp. 1** | **Comp. 2** | **Comp. 3** | **Ex. 2** |
|---|---|---|---|---|---|
| **SM stream (190) Units** | 100.0 | 100.0 | 101.8 | 102.0 | 99.8 |
| **Component** | | | | | |
| Styrene Monomer (%wt) | 99.96 | 99.97 | 99.90 | 99.85 | 99.96 |
| ethylbenzene (ppm) | 98 | 98 | 231 | 264 | 97 |
| cumene (ppm) | 25 | 4 | 92 | 125 | 25 |
| alpha-methyl-styrene (ppm) | 93 | 16 | 155 | 443 | 89 |
| C8 aromatic compounds^{(a)} | 26 | 8 | 255 | 256 | 26 |
| phenylacetylene (ppm) | 0 | 0 | 19 | 21 | 0 |
| allyphatic c6-c8 hc's (ppm) | 0 | 0 | 102 | 109 | 0 |
| C9 aromatic compounds^{(b)} | 6 | 4 | 29 | 47 | 6 |

| | | | | | |
|---|---|---|---|---|---|
| (a) a mixture comprising ortho-xylene, meta-xylene, and para-xylene, among others; (b) a mixture comprising propenylbenzene, n-propylbenzene; allyl-benzene, ethyl-toluene and methyl-styrene, among others. | | | | | |

Some specification values for Styrene Monomer quality are (see PERP0304-8 Styrene-Ethylbenzene, page 39)

| Styrene | 99.90 wt% min |
|---|---|
| Aldehydes (as benzaldehyde) | 50 wppm max |
| Peroxides (as H₂O₂) | 20 wppm max |
| Polymer | 5 wppm max |
| Alpha-Methyl Styrene | 250 wppm max |
| C8/C9 aromatics | 100 wppm max |

C₈/C₉ aromatics would include: o-, m-, p-xylenes, ethylbenzene, phenylacetylene, allylbenzene, n-propylbenzene, methyl-ethylbenzenes and methylstyrenes.

A typical analysis of commercially available styrene (see Safe Handling and storage of Styrene Monomer de Chevron Phillips, 20-8-2009, page 8)

| Component | Value | Units |
|---|---|---|
| Styrene | 99.93 | %wt |
| Benzene | <1 | ppm |
| toluene | <1 | ppm |
| Ethylbenzene | 50 | ppm |
| alpha-methylstyrene | 175 | ppm |
| m+p-xylene | 120 | ppm |
| o-xylene | 125 | ppm |
| cumene | 100 | ppm |
| n-propylbenzene | 60 | ppm |
| m+p-methylethylbenzene | 20 | ppm |
| methylstyrene | 10 | ppm |
| phenylacetylene | 50 | ppm |
| m+p-divinylbenzene | <10 | ppm |
| o-divinylbenzene | <5 | ppm |
| polymer | 1 | ppm |
| TBC | 12 | ppm |
| aldehydes as benzaldehyde | 15 | ppm |
| peroxides as benzoylperoxides | 5 | ppm |
| chlorides as Cl | < 1 | ppm |
| Sulfur as S | < 1 | ppm |

The following conclusions can be achieved from the examples above:
- SM richness. Comparative 2 remains in limit and comparative 3 would be out of specification, while the rest of the examples are above the lower specification limit, being the value of Example 1 and Example 2 similar to comparative 1 (reference).
- Alpha-methylstyrene: in comparative 3 would be above the reference specification and the in comparative 2 is increased over the reference example (comparative 1) and Example 1 and Example 2, where the alpha-methylstyrene content is higher than comparative 1, but within the order of value.
- C8/C9 aromatics. All examples show values above the reference specification (up to comparative 1 which is reference example without the oil) but, in Example 1 and Example 2 although having a slightly higher value, this is very similar to comparative 1 (reference) and in examples comparative 2 and comparative 3, the values increase very substantially. It is observed that in Example 1 and Example 2 (in addition to comparative1) the values are better than a the typical analysis of commercially available styrene showed before and in comparative 2 and comparative 3 the result is worse, particularly in the EB content.
- The C6/C8 aliphatics would be new components that do not appear in the current commercial SM, with potential affectation to the commercial uses of styrene and, that in the comparative 2 and comparative 3 are present in the SM product while in the rest of the cases they are not.

As observed, examples 1 and 2, where pyrolysis oil is previously hydrogenated, there is no net increase in SM production (what is achieved is to "produce" EB). In the comparative cases 2 and 3 there is an increase in production. Further, in examples 1 and 2, although there is obserbed a net increase in xylenes, cumene and alpha-methylstyrene, they are within ASTM D2827 specifications. Whereas in comparative examples 2 and 3, the product does not meet with ASTM D2827 specifications for these components.

Additionally, there is a significant number of impurities present in SM product as obtained in comparative examples 2 and 3.

Xylene containing product stream (130) comprises the composition as disclosed in Table 13.

**Table 13. Xylene containing product stream (130) composition**

| | **Ex 1** | **Comp. 1** | **Comp. 2** | **Comp. 3** | **Ex. 2** |
|---|---|---|---|---|---|
| **Production t / t SM (190)*** | 0.00656 | 0.00682 | 0.00659 | 0.00632 | 0.00646 |

| **Component** | **t / t SM (190)**** | | | | |
|---|---|---|---|---|---|
| Ethylbenzene | 0.000370 | 0.000359 | 0.000358 | 0.000366 | 0.000365 |
| C8 aromatic compounds^{a} | 0.000301 | 0.000093 | 0.0001 | 0.0001 | 0.000292 |
| Styrene | 0.000102 | 0.000100 | 0.000100 | 0.000100 | 0.000102 |
| Cumene | 0.000302 | 0.000042 | 0.000223 | 0.000610 | 0.000301 |
| Alpha-methyl-styrene | 0.000000 | 0.000000 | 0.000000 | 0.000000 | 0.000000 |
| Benzaldehyde | 0.002899 | 0.002912 | 0.002844 | 0.002776 | 0.002904 |
| C9 aromatic compounds^{b} | 0.000132 | 0.000075 | 0.000103 | 0.000416 | 0.000129 |
| C10 Aromatic compounds^{c} | 0.000041 | 0.000041 | 0.000040 | 0.000040 | 0.000041 |
| 1,2-propanediol | 0.001182 | 0.001432 | 0.001305 | 0.000992 | 0.001151 |
| Acetophenone | 0.000253 | 0.000485 | 0.000375 | 0.000086 | 0.000234 |
| 1-phenylethanol | 0.000005 | 0.000008 | 0.000007 | 0.000002 | 0.000003 |
| Heavies^{d} | 0.000973 | 0.001266 | 0.001135 | 0.000573 | 0.000932 |

| | | | | | |
|---|---|---|---|---|---|
| * "Production t/t SM (190)" means xylene containing product stream (130) per Styrene Monomer Product stream (190) mass ratio in tonnes per tonnes ** "t/t SM product" means metric tonnes of product per metric tonnes of styrene monomer stream (190) (a) a mixture comprising ortho-xylene, meta-xylene, and para-xylene, among others; (b) a mixture comprising n-propylbenzene and ethyl-toluene (c) mainly diethylbenzene (d) mainly styrene dimer | | | | | |

It can be seen that in examples 1 and 2, for the same total production, there is greater effective purge of xylenes, isopropylbenzene (cumene) and n-propylbenzene compared to comparative example 1, so that it is proven that impurities are effectively removed by this purge on the basis of the invention.

In conclusion, feeding of pyrolysis oil as according to comparative example 2 or 3 is not feasible.

Direct input of a polystyrene pyrolyisis oil, comparative example 3, or a C8 fraction obtained by distilling polystyrene pyrolysis oil, comparative example 2, to the styrene purification section of the PO/SM process means direct incorporation of EB, cumene, n-propylbenzene, AMS and xylenes (among others) into the SM product, taking it out of specification.. Additionally, "new" impurities are incorporated into the SM (C7-C8 aliphatic hydrocarbons, allylbenzene, ethyltoluene).

No useful process variable is available to control the above values, beyond restricting their entry into the pyrolysis oil if possible, limiting possibilites of use available pyrolitic processes or, employing complex purification processes which require a very high number of separation steps by distillation and/or amounts of energy reducing or eliminitaing process integration benefits in energy comsuption ).

According to example 1, the net production of SM would be maintained, but around 0,017 t EB / t SM of "virgin" EB (100) would be saved by "circular" ethylbenzene generated from the pyrolysis oil as per example showed, generating and equivalent of SM of "circular" origin as a consecuence of polystyrene pyrolysis oil incorporation into the process (the quality of the SM remains within specifications).

According to comparative example 2, the fractionated styrene product stream (30) fed (pre-fractionated) to the process through the feed to distillation column (D5). Being a pre-fractionated feed (weights have been eliminated), it implies an "extra" load to the distillation section of the hydrogenation unit.

### References cited in the application

1. ASTM D-2827
2. Ki-bum Park et al. Applied Energy 259, 2020, pp. 114240
3. Ibrahim M. Maafa. Polymers, 2021, vol. 13, pp. 225
4. John Scheirs, and Walter Kaminsky. "Feedstock Recycling and Pyrolysis of Waste Plastics: Converting Waste Plastics into Diesel and Other Fuels", Wiley Online Library 2006, p635
5. Jasmin Shah et al. "Conversion of waste polystyrene through catalytic degradation into valuable products", Korean J. Chem. Eng., 2014, vol. 31(8), pp.1389-1398.
6. ASTM D3193-17
7. ASTM D664
8. EPA 3051a
9. ASTM D6052-97.
10. ASTM D2827
11. US10301235
12. WO2021230312
13. WO2021053074.
14. PERP 2012-7 Propylene Oxide, pages 19-26
15. PERP0304-8 Styrene-Ethylbenzene, page 39
16. Safe Handling and storage of Styrene Monomer de Chevron Phillips, 20-8-2009, page 8

## Claims

1. A method comprising:
a) providing a hydrocarbon liquid stream (10) resulting from the pyrolysis of waste plastic;
b) introducing the hydrocarbon liquid stream (10) into
b.1) a first fractional distillation unit (T1) to separate a C7 stream overhead (40), a first fractionated sidestream (30), a heavies-containing stream (20) from bottom, and, optionally, an alpha-methyl-styrene containing product sidestream (50);
alternatively,
b.2) a first fractional distillation unit (T1) to separate a C7 stream overhead (40) and a stream which is introduced into a second fractional distillation unit (T1') obtaining a first fractionated sidestream (30), a heavies containing stream (20) from bottom, and, optionally, an alpha-methyl-styrene containing product sidestream (50),
or alternatively
b.3) a distillation column (D5), thus obtaining a distilled out product stream (220);
c) feeding
c.1) the first fractionated sidestream (30) obtained in b.1) or b.2), or the distilled out product stream (220) obtained in b.3), to a hydroprocessing unit (HP) comprising an hydroprocessing catalyst in the presence of hydrogen, to yield a ethylbenzene containing product stream (80, 270);
or alternatively,
c.2) the first fractionated sidestream (30) obtained in b.1) or b.2), to a distillation column (D5), obtaining a distilled out product stream (220); the distilled out product stream (220) is then fed to the hydroprocesing unit (HP) comprising an hydroprocessing catalyst in the presence of hydrogen to yield a ethylbenzene containing product stream (270);
d) incorporating the ethylbenzene containing product stream (80, 270) thus obtained in c.1) or c.2), to a ethylbenzene distillation column (D1), thus a distilled ethylbenzene stream (110) is recovered from the top of the tower and an alpha-methylbenzyl alcohol containing stream (120) is recovered from bottoms;
e) incorporating the ethylbenzene product stream (110), optionally together with fresh ethylbenzene (100), to a ethylbenzene oxidation unit to produce ethylbenzene hydroperoxide by oxidation in liquid phase;
f) reacting the ethylbenzene hydroperoxide obtained in e) with a propylene stream in an epoxidation unit in the presence of a catalyst to produce crude propylene oxide (300) and a bottoms product (310) containing ethylbenzene and alpha-methylbenzyl alcohol ;
g) feeding the alpha-methylbenzyl alcohol containing stream (120) to a second distillation column (D2), thus obtaining three fractions: a xylene containing product stream overhead (130), a alpha-methylbenzyl alcohol rich sidestream (150), and a bottom stream (140) containing alpha-methylbenzyl alcohol and higher boiling point products;
or alternatively, the alpha-methylbenzyl alcohol containing stream (120) is introduced into a second distillation column (D2) to separate a xylene containing stream product overhead (130), and a bottom stream which is introduced into an additional distillation column (D2'), thus obtaining a alpha-methylbenzyl alcohol rich top stream (150), and a heavy bottom stream (140) containing alpha-methylbenzyl alcohol and higher boiling point products;
h) incorporating the alpha-methylbenzyl alcohol rich sidestream (150) to a dehydration unit (DH1) containing a catalyst, thus obtaining crude styrene monomer (160) and water, which are phase separated;
i) feeding the crude styrene phase (160) thus obtained to a distillation column (D3) wherein a ethylbenzene containing stream (170) is top separated, and the bottom product (180) is fed to a styrene product column (D4) where styrene monomer is obtained at the top (190), and a heavy product at the bottom (200);
j) feeding the heavy bottom product streams (140 and 200), resulting from the second and the additional distillation columns (D2, D2') and styrene product column (D4), to the distillation column (D5); the distilled out product stream (220) thus obtained is fed alongside the ethylbenzene containing stream (170) to the hydroprocesing unit (HP) of step c).

2. The method according to claim 1, wherein the first fractionated sidestream (30) obtained following either option b1) or option b2), comprise a benzene content of from 0 to 250 ppmw.

3. The method according to any of claims 1-2, wherein the first fractionated sidestream (30) obtained following either option b1) or option b2), comprise a styrene and ethylbenzene content from 89 wt% to 99 wt% in relation to the total weight of the stream.

4. The method according to any of claims 1-3, wherein the first fractionated pyrolysis oil (30) sidestream obtained following either option b1) or option b2), comprise from 0.01 to 1 mgKOH/g of acid containing compounds measured by the standard test method ASTM D664

5. The method according to any of claims 1-4, wherein the first fractionated sidestream (30) obtained following either option b1) or option b2), comprises
i. from 89 to 99 % by weight of styrene monomer plus ethylbenzene, preferably from 90 to 98% by weight, more preferably from 93 to 95% by weight;
ii. from 0 to 500 ppm by weight of benzene, preferably from 50 to 300 ppm by weight, more preferably from 100 to 250 ppm by weight;
iii. from 0 to 1 % by weight of toluene, preferably from 0.01 to 0.5% by weight, more preferably from 0.1 to 0.3% by weight;
iv. from 0 to 2.5 % by weight of xylenes, preferably from 0.1 to 2% by weight, more preferably from 0.5 to 1.5% by weight;
v. from 0 to 1.5 % by weight of C9 aromatic compounds others than alpha-methyl styrene a mixture of cumene; propenyl benzene, n-propylbenzene; allyl-benzene, ethyl-toluene and methyl-styrene, preferably from 0.01 to 1% by weight, more preferably from 0.1 to 0.8% by weight;
vi. from 0 to 5 % by weight of alpha-methyl styrene, preferably from 0.1 to 3% by weight, more preferably from 1 to 2.5% by weight;
vii. from 0 to 1.5 % by weight of phenylacetylene preferably from 0.0001 to 1% by weight, more preferably from 0.001 to 0.5% by weight;
viii. from 0 to 2500 ppm by weight of heteroatom containing compounds , preferably from 1 to 1250 ppm by weight, more preferably from 10 to 250 ppm by weight;
ix. from 0 to 0.1% by weight of C10 aromatic compounds a mixture of divinylbenzene and ethyl-styrene, preferably from 0.001 to 0.08% by weight, more preferably from 0.01 to 0.05% by weight;
x. from 0 to 1% by weight of one or more compounds selected from the group consisting of a mixture of C3-C8 alkanes, C2-C8 alkenes, C5-C8 cycloalkanes and C5-C8 cycloalkenes, preferably from 0.001 to 0.85% by weight, more preferably from 0.01 to 0.5% by weight;
xi. from 0 to 0.1% by weight of a heavy oil fraction, preferably from 0.0001 to 0.05% by weight, more preferably from 0.001 to 0.01% by weight;
xii. from 0 to 50 ppm by weight of metals, of metal containing compounds, preferably from 0.01 to 25 ppm by weight, more preferably from 0.1 to 10 ppm by weight;
xiii. from 0.5 to 1500 ppm by weight of heteroatom, of heteroatom containing compounds preferably from 1 to 500 ppm by weight, more preferably from 5 to 100 ppm by weight; and
xiv. from 0 to 120 ppm by weight of water, preferably from 0.1 to 25 ppm by weight, more preferably from 1 to 10 ppm by weight.

6. The method according to any of claims 1-5, wherein, when following either option b.1) or b.2), previously to step c), the method further comprises an alkaline or aqueous washing of the first fractionated pyrolysis oil sidestream (30).

7. The method according to any of claims 1-6, wherein the first fractionated pyrolysis oil sidestream (30) obtained in b.1) or b.2) is introduced into an hydroprocessing unit (HP) comprising an heterogeneous hydroprocessing catalyst in the presence of hydrogen according to step c.1); and wherein the hydroprocessing unit (HP) is selected from
c.1A) a dedicated hydroprocessing unit (HP2);
and
c.1B) an hydroprocessing unit (HP1) used in an acetophenone hydrogenation step,wherein the first fractionated sidestream (30) is introduced into the hydroprocessing unit (HP1) together with the distilled out product stream of a distillation column (D5).

8. The method according to claim 7, wherein the hydroprocessing unit (HP) of step c.1B) comprises two or more slurry hydrogenation reactors (HP1₁, HP1₂,... HP1ₙ) connected in series with each other; wherein the first fractionated sidestream (30) is distributed between the different HP1ₙ reactors; and wherein the portion of the first fractionated sidestream (30) fed to the first hydroprocessing reactor (HP1₁), yields an ethylbenzene containing product stream which is fed into the immediately subsequent reactor, and then this action is repeated for a number of n reactors until the nth hydroprocessing reactor (HP1ₙ), to yield a ethylbenzene containing product stream (270).

9. The method according to claim 8, wherein the first fractionated sidestream (30) is distributed between the different HP1ₙ reactors at an styrene monomer concentration lower than 15 wt% based on the total fed stream of each reactor.

10. The method according to any of claims 1-6, wherein in step c), the first fractionated sidestream (30) is fed to a distillation column (D5) together with the heavy bottom product streams (140) and (200), obtaining a distilled out product stream (220); where the distilled out product stream (220) is fed to an hydroprocessing unit (HP1) used in an acetophenone hydrogenation step.

11. The method according to any of claims 1-10, which further comprises converting a heavy residue containing styrene oligomers (155), which is obtained in the dehydration unit (DH1), in a pyrolysis unit, to a recycled pyrolysis oil stream (10'); and introducing the recycled pyrolysis oil stream (10') thus obtained to the first fractional distillation unit (T1) together with the hydrocarbon liquid stream resulting from the pyrolysis of waste plastic (10) or alternatively to the distillation column (D5).

12. The method according to any of claims 1-11, wherein the distilled ethylbenzene stream (110) obtained in step d) comprises
from 93 to 99 wt% of ethylbenzene, preferably from 95 to 99 wt%;
from 0.001 to 0.1 wt% of benzene, preferably from 0.01 to 0.05 wt%;
from 0.01 to 1 wt% of toluene, preferably from 0.1 to 0.5 wt%;
from 0.01 to 1 wt% of o-xylene, preferably from 0.1 to 0.5 wt%;
from 0.01 to 3 wt% of m-xylene, preferably from 0.1 to 1.5 wt%;
from 0.01 to 3 wt% of p-xylene, preferably from 0.1 to 1.5 wt%;
from 0.01 to 1 wt% of isopropylbenzene, preferably from 0.1 to 0.5 wt%;
from 1 to 1000 ppm wt of diethylbenzene, preferably from 10 to 100 ppm wt;
from 0.0001 to 0.1 %wt of n-propylbenzene, preferably from 0.001 to 0.01 wt%;
from 0.001 to 0.1 %wt of one or more compounds selected from the group consisting of a mixture of C3-C8 alkanes, C2-C8 alkenes, C5-C8 cycloalkanes and C5-C8 cycloalkenes, preferably from 0.01 to 0.06 wt%;
from 0.01 to 50 ppmw of heteroatom containing compounds, preferably from 0.1 to 5 ppmw;.and
from 0.1 to 250 ppmw of water, preferably from 0.1 to 100 ppmw.

## Patentansprüche

1. Ein Verfahren, umfassend:
a) bereitstellen eines flüssigen Kohlenwasserstoffstroms (10), der aus der Pyrolyse von Kunststoffabfällen resultiert;
b) einleiten des flüssigen Kohlenwasserstoffstroms (10) in
b.1) eine erste fraktionelle Destillationseinheit (T1), um einen C7-Kopfproduktstrom (40), einen ersten fraktionierten Seitenstrom (30), einen hochsiedende Stoffe enthaltenden Strom (20) vom Sumpf und, wahlweise, einen alpha-Methylstyrol enthaltenden Produktseitenstrom (50) zu trennen; ersatzweise,
b.2) eine erste fraktionelle Destillationseinheit (T1), um einen Kopfproduktstrom C7 (40) und einen Stroms, der in eine zweite fraktionelle Destillationseinheit (T1') eingeleitet wird, zu trennen, wodurch ein erster fraktionierter Seitenstrom (30), ein hochsiedende Stoffe enthaltender Strom (20) aus dem Sumpf und, wahlweise, ein alpha-Methylstyrol enthaltender Produktseitenstrom (50) erhalten werden,
oder ersatzweise
b.3) eine Destillationskolonne (D5), wodurch ein abdestillierter Produktstrom (220) erhalten wird;
c) einspeisen von
c.1) dem in b.1) oder b.2) erhaltenen ersten fraktionierten Seitenstrom (30) oder dem in b.3) erhaltenen abdestillierten Produktstrom (220) in eine Hydroprocessing-Einheit (HP), die einen Hydroprocessing-Katalysator in Gegenwart von Wasserstoff umfasst, um einen Ethylbenzol enthaltenden Produktstrom (80, 270) zu erhalten;
oder ersatzweise,
c.2) dem in b.1) oder b.2) erhaltenen ersten fraktionierten Seitenstrom (30) in eine Destillationskolonne (D5), wodurch ein abdestillierter Produktstrom (220) erhalten wird; der abdestillierte Produktstrom (220) wird dann der Hydroprocessing-Einheit (HP) eingespeist, die einen Hydroprocessing-Katalysator in Gegenwart von Wasserstoff umfasst, um einen Ethylbenzol enthaltenden Produktstrom (270) zu erhalten;
d) einfließen lassen des somit in c.1) oder c.2) erhaltenen Ethylbenzol enthaltenden Produktstroms (80, 270) in eine Ethylbenzol-Destillationskolonne (D1), wodurch ein destillierter Ethylbenzolstrom (110) am oberen Teil des Turms und ein alpha-Methylbenzylalkohol enthaltender Strom (120) aus dem Sumpf gewonnen werden;
e) einfließen lassen des Ethylbenzolproduktstroms (110), wahlweise zusammen mit frischem Ethylbenzol (100), in eine Ethylbenzoloxidationseinheit, um Ethylbenzolhydroperoxid durch Oxidation in flüssiger Phase herzustellen;
f) umsetzen des in e) erhaltenen Ethylbenzolhydroperoxids mit einem Propylenstrom in einer Epoxidierungseinheit in Gegenwart eines Katalysators, um rohes Propylenoxid (300) und ein Sumpfprodukt (310), das Ethylbenzol und alpha-Methylbenzylalkohol enthält, herzustellen;
g) einspeisen des alpha-Methylbenzylalkohol enthaltenden Stroms (120) in eine zweite Destillationskolonne (D2), wodurch drei Fraktionen erhalten werden: ein Xylol enthaltender Kopfproduktstrom (130), ein an alpha-Methylbenzylalkohol reicher Seitenstrom (150) und ein Sumpfstrom (140), der alpha-Methylbenzylalkohol und Produkte mit höherem Siedepunkt enthält; oder ersatzweise wird der alpha-Methylbenzylalkohol enthaltende Strom (120) in eine zweite Destillationskolonne (D2) eingeleitet, um einen Xylol enthaltenden Kopfproduktstrom (130) und einen Sumpfstrom, der in eine zusätzliche Destillationskolonne (D2') eingeleitet wird, zu trennen, wodurch ein an alpha-Methylbenzylalkohol reicher oberer Strom (150) und ein alpha-Methylbenzylalkohol und Produkte mit höherem Siedepunkt enthaltender hochsiedender Sumpfstrom (140) erhalten werden;
h) einfließen lassen des an alpha-Methylbenzylalkohol reichen Seitenstroms (150) in eine Dehydratisierungseinheit (DH1), die einen Katalysator enthält, wodurch rohes Styrolmonomer (160) und Wasser erhalten werden, welche phasengetrennt werden;
i) einspeisen der somit erhaltenen rohen Styrolphase (160) in eine Destillationskolonne (D3), wobei ein Ethylbenzol enthaltender Strom (170) von oben abgetrennt wird und das Sumpfprodukt (180) in eine Styrolproduktkolonne (D4) eingespeist wird, wo Styrolmonomer oben (190) und ein hochsiedendes Produkt am Sumpf (200) erhalten wird;
j) einspeisen der hochsiedenden Sumpfproduktströme (140 und 200), die aus der zweiten und den zusätzlichen Destillationskolonnen (D2, D2') und Styrolproduktkolonne (D4) resultieren, in die Destillationskolonne (D5); der somit erhaltene abdestillierte Produktstrom (220) wird neben dem Ethylbenzol enthaltenden Strom (170) in die Hydroprocessing-Einheit (HP) von Schritt c) eingespeist.

2. Das Verfahren nach Anspruch 1, wobei der erste fraktionierte Seitenstrom (30), der entweder nach Option b1) oder Option b2) erhalten wird, einen Benzolgehalt von 0 bis 250 ppmw enthält.

3. Das Verfahren nach einem der Ansprüche 1 bis 2, wobei der erste fraktionierte Seitenstrom (30), der entweder nach Option b1) oder Option b2) erhalten wird, einen Styrol- und Ethylbenzolgehalt von 89 Gew.-% bis 99 Gew.-% in Bezug auf das Gesamtgewicht des Stroms umfasst.

4. Das Verfahren nach einem der Ansprüche 1 bis 3, wobei der erste fraktionierte Pyrolyseöl entsprechender (30) Seitenstrom, der entweder nach der Option b1) oder der Option b2) erhalten wird, 0,01 bis 1 mg KOH/g säurehaltige Verbindungen, gemessen nach der Standardtestmethode ASTM D664, umfasst.

5. Das Verfahren nach einem der Ansprüche 1 bis 4, wobei der erste fraktionierte Seitenstrom (30), der entweder nach Option b1) oder Option b2) erhalten wird, Folgendes umfasst:
i. von 89 bis 99 Gew.- % Styrolmonomer plus Ethylbenzol, bevorzugt von 90 bis 98 Gew.- %, stärker bevorzugt von 93 bis 95 Gew.- %;
ii. von 0 bis 500 Gew.-ppm Benzol, bevorzugt von 50 bis 300 Gew.-ppm, stärker bevorzugt von 100 bis 250 Gew.-ppm;
iii. von 0 bis 1 Gew.- % Toluol, bevorzugt von 0,01 bis 0,5 Gew.- %, stärker bevorzugt von 0,1 bis 0,3 Gew.- %;
iv. von 0 bis 2,5 Gew.- % Xylole, bevorzugt von 0,1 bis 2 Gew.- %, stärker bevorzugt von 0,5 bis 1,5 Gew.- %;
v. von 0 bis 1,5 Gew.- % C9-Aromaten, außer alpha-Methylstyrol, eines Gemisches aus Cumol; Propenylbenzol, n-Propylbenzol; Allylbenzol, Ethyltoluol und Methylstyrol, bevorzugt von 0,01 bis 1 Gew.- %, stärker bevorzugt von 0,1 bis 0,8 Gew.- %;
vi. von 0 bis 5 Gew.- % alpha-Methylstyrol, bevorzugt von 0,1 bis 3 Gew.-%, stärker bevorzugt von 1 bis 2,5 Gew.- %;
vii. von 0 bis 1,5 Gew.- % Phenylacetylen, bevorzugt von 0,0001 bis 1 Gew.- %, stärker bevorzugt von 0,001 bis 0,5 Gew.- %;
viii. von 0 bis 2500 Gew.-ppm heteroatomhaltige Verbindungen, bevorzugt von 1 bis 1250 Gew.-ppm, stärker bevorzugt von 10 bis 250 Gew.-ppm;
ix. von 0 bis 0,1 Gew.- % C10-Aromaten eines Gemisches aus Divinylbenzol und Ethylstyrol, bevorzugt von 0,001 bis 0,08 Gew.- %, stärker bevorzugt von 0,01 bis 0,05 Gew.- %;
x. von 0 bis 1 Gew.- % einer oder mehrerer Verbindungen ausgewählt aus der Gruppe bestehend aus einer Mischung von C3-C8-Alkanen, C2-C8-Alkenen, C5-C8-Cycloalkanen und C5-C8-Cycloalkenen, bevorzugt von 0,001 bis 0,85 Gew.- %, stärker bevorzugt von 0,01 bis 0,5 Gew.- %;
xi. von 0 bis 0,1 Gew.- % einer Schwerölfraktion, bevorzugt von 0,0001 bis 0,05 Gew.- %, stärker bevorzugt von 0,001 bis 0,01 Gew.- %;
xii. von 0 bis 50 Gew.-ppm Metallen, an metallhaltigen Verbindungen, bevorzugt von 0,01 bis 25 Gew.-ppm, stärker bevorzugt von 0,1 bis 10 Gew.-ppm;
xiii. von 0,5 bis 1500 Gew.-ppm Heteroatom, von heteroatomhaltigen Verbindungen, bevorzugt von 1 bis 500 Gew.-ppm, stärker bevorzugt von 5 bis 100 Gew.-ppm; und
xiv. von 0 bis 120 Gew.-ppm Wasser, bevorzugt von 0,1 bis 25 Gew.-ppm, stärker bevorzugt von 1 bis 10 Gew.-ppm.

6. Das Verfahren nach einem der Ansprüche 1 bis 5, wobei, wenn entweder Option b.1) oder b.2) gefolgt wird, das Verfahren vor dem Schritt c) ferner ein alkalisches oder wässriges Waschen des ersten fraktionierten Pyrolyseöl-Seitenstroms (30) umfasst.

7. Das Verfahren nach einem der Ansprüche 1 bis 6, wobei der erste fraktionierte Pyrolyseöl-Seitenstrom (30), der in b.1) oder b.2) erhalten wird, in eine Hydroprocessing-Einheit (HP) eingeleitet wird, die einen heterogenen Hydroprocessing-Katalysator in Gegenwart von Wasserstoff gemäß Schritt c.1) umfasst; und wobei die Hydroprocessing-Einheit (HP) ausgewählt ist aus
c.1A) einer dedizierten Hydroprocessing-Einheit (HP2);
und
c.1B) einer in einem Acetophenon-Hydrierschritt verwendeten Hydroprocessing-Einheit (HP1), wobei der erste fraktionierte Seitenstrom (30) zusammen mit dem abdestillierten Produktstrom einer Destillationskolonne (D5) in die Hydroprocessing-Einheit (HP1) eingeleitet wird.

8. Das Verfahren nach Anspruch 7, wobei die Hydroprocessing-Einheit (HP) von Schritt c.1B) zwei oder mehr Aufschlämmungshydrierungsreaktoren (HP1₁, HP1 ₂, ...HP1ₙ) umfasst, die in Reihe miteinander verbunden sind; wobei der erste fraktionierte Seitenstrom (30) auf die verschiedenen HP1ₙ Reaktoren verteilt ist; und wobei der Teil des ersten fraktionierten Seitenstroms (30), der in den ersten Hydroprocessing-Reaktor (HP1₁) eingespeist wird, einen Ethylbenzol enthaltenden Produktstrom ergibt, der in den unmittelbar nachfolgenden Reaktor eingespeist wird, und wobei dieser Vorgang dann für eine Anzahl von n Reaktoren bis zum n-ten Hydroprocessing-Reaktor (HP1ₙ) wiederholt wird, um einen Ethylbenzol enthaltenden Produktstrom (270) zu ergeben.

9. Das Verfahren nach Anspruch 8, wobei der erste fraktionierte Seitenstrom (30) auf die verschiedenen HP1ₙ Reaktoren bei einer Styrolmonomerkonzentration von weniger als 15 Gew.-%, bezogen auf den gesamten eingespeisten Strom jedes Reaktors, verteilt wird.

10. Das Verfahren nach einem der Ansprüche 1 bis 6, wobei in Schritt c) der erste fraktionierte Seitenstrom (30) zusammen mit den hochsiedenden Sumpfproduktströmen (140) und (200) einer Destillationskolonne (D5) eingespeist wird, wodurch ein abdestillierter Produktstrom (220) erhalten wird; wobei der abdestillierte Produktstrom (220) in eine Hydroprocessing-Einheit (HP1) eingespeist wird, die in einem Acetophenonhydrierschritt verwendet wird.

11. Das Verfahren nach einem der Ansprüche 1 bis 10, das ferner das Umwandeln eines schweren Rückstands, der Styrololigomere (155) enthält, der in der Dehydratisierungseinheit (DH1) erhalten werden, in einer Pyrolyseeinheit, in einen recycelten Pyrolyseölstrom (10') und das Einleiten des somit erhaltenen recycelten Pyrolyseölstroms (10') in die erste fraktionelle Destillationseinheit (T1) zusammen mit dem flüssigen Kohlenwasserstoffstrom, der aus der Pyrolyse von Kunststoffabfällen (10) resultiert, oder ersatzweise in die Destillationssäule (D5) umfasst.

12. Das Verfahren nach einem der Ansprüche 1 bis 11, wobei der in Schritt d) erhaltene destillierte Ethylbenzolstrom (110) Folgendes umfasst
von 93 bis 99 Gew.-% Ethylbenzol, bevorzugt von 95 bis 99 Gew.-%;
von 0,001 bis 0,1 Gew.-% Benzol, bevorzugt von 0,01 bis 0,05 Gew.-%;
von 0,01 bis 1 Gew.-% Toluol, bevorzugt von 0,1 bis 0,5 Gew.-%;
von 0,01 bis 1 Gew.-% o-Xylol, bevorzugt von 0,1 bis 0,5 Gew.-%;
von 0,01 bis 3 Gew.-% m-Xylol, bevorzugt von 0,1 bis 1,5 Gew.-%;
von 0,01 bis 3 Gew.-% p-Xylol, bevorzugt von 0,1 bis 1,5 Gew.-%;
von 0,01 bis 1 Gew.-% Isopropylbenzol, bevorzugt von 0,1 bis 0,5 Gew.-%;
von 1 bis 1000 Gew.-ppm Diethylbenzol, bevorzugt von 10 bis 100 Gew.-ppm; von 0,0001 bis 0,1 Gew.- % n-Propylbenzol, bevorzugt von 0,001 bis 0,01 Gew.-%;
von 0,001 bis 0,1 Gew.- % einer oder mehrerer Verbindungen, die aus der Gruppe ausgewählt sind, die aus einer Mischung von C3-C8-Alkanen, C2-C8-Alkenen, C5-C8-Cycloalkanen und C5-C8-Cycloalkenen besteht, bevorzugt von 0,01 bis 0,06 Gew.-%;
von 0,01 bis 50 ppmw heteroatomhaltiger Verbindungen, bevorzugt von 0,1 bis 5 ppmw; und
von 0,1 bis 250 ppmw Wasser, bevorzugt von 0,1 bis 100 ppmw.

## Revendications

1. Un procédé comprenant :
a) fournir un flux liquide d'hydrocarbures (10) résultant de la pyrolyse de déchets plastiques ;
b) introduire le flux liquide d'hydrocarbures (10) dans
b.1) une première unité de distillation fractionnée (T1) pour séparer un flux de tête C7 (40), un premier flux latéral fractionné (30), un flux contenant des fractions lourdes (20) du fond et, facultativement, un flux latéral de produit contenant de l'alpha-méthyl-styrène (50) ;
en variante,
b.2) une première unité de distillation fractionnée (T1) pour séparer un flux de tête C7 (40) et un flux qui est introduit dans une seconde unité de distillation fractionnée (T1') obtenant un premier flux latéral fractionné (30), un flux contenant des produits lourds (20) de fond et, facultativement, un flux latéral de produit contenant de l'alpha-méthyl-styrène (50),
ou, en variante,
b.3) une colonne de distillation (D5), obtenant ainsi un flux de produit distillé (220) ;
c) alimenter
c.1) le premier flux latéral fractionné (30) obtenu en b.1) ou b.2), ou le flux de produit distillé (220) obtenu en b.3), à une unité d'hydrotraitement (HP) comprenant un catalyseur d'hydrotraitement en présence d'hydrogène, pour donner un flux de produit contenant de l'éthylbenzène (80, 270) ;
ou, en variante,
c.2) le premier flux latéral fractionné (30) obtenu en b.1) ou b.2), à une colonne de distillation (D5), obtenant un flux de produit distillé (220) ; le flux de produit distillé (220) est puis alimenté à l'unité d'hydrotraitement (HP) comprenant un catalyseur d'hydrotraitement en présence d'hydrogène pour donner un flux de produit contenant de l'éthylbenzène (270) ;
d) incorporer le flux de produit contenant de l'éthylbenzène (80, 270) ainsi obtenu en c.1) ou c.2), dans une colonne de distillation d'éthylbenzène (D1), ainsi un flux d'éthylbenzène distillé (110) est récupéré à partir du sommet de la tour et un flux contenant de l'alcool alpha-méthylbenzylique (120) est récupéré à partir du produit de bas de colonne ;
e) incorporer le flux de produit d'éthylbenzène (110), facultativement avec de l'éthylbenzène frais (100), à une unité d'oxydation d'éthylbenzène pour produire de l'hydroperoxyde d'éthylbenzène par oxydation en phase liquide ;
f) faire réagir l'hydroperoxyde d'éthylbenzène obtenu en e) avec un flux de propylène dans une unité d'époxydation en présence d'un catalyseur pour produire de l'oxyde de propylène brut (300) et un produit de bas de colonne (310) contenant de l'éthylbenzène et de l'alcool alpha-méthylbenzylique ;
g) alimenter le flux contenant de l'alcool alpha-méthylbenzylique (120) dans une seconde colonne de distillation (D2), obtenant ainsi trois fractions : un flux de produit de tête contenant du xylène (130), un flux latéral riche en alcool alpha-méthylbenzylique (150) et un flux de fond (140) contenant de l'alcool alpha-méthylbenzylique et des produits à point d'ébullition plus élevé ;
ou, en variante, le flux contenant de l'alcool alpha-méthylbenzylique (120) est introduit dans une seconde colonne de distillation (D2) pour séparer un flux de produit de tête contenant du xylène (130), et un flux de fond qui est introduit dans une colonne de distillation supplémentaire (D2'), obtenant ainsi un flux de sommet riche en alcool alpha-méthylbenzylique (150), et un flux de fond lourd (140) contenant de l'alcool alpha-méthylbenzylique et des produits à point d'ébullition plus élevé ;
h) incorporer le flux latéral riche en alcool alpha-méthylbenzylique (150) à une unité de déshydratation (DH1) contenant un catalyseur, obtenant ainsi un monomère de styrène brut (160) et de l'eau, qui sont séparés en phases ;
i) alimenter la phase de styrène brut (160) ainsi obtenue à une colonne de distillation (D3) dans laquelle un flux contenant de l'éthylbenzène (170) est séparé par le haut, et le produit de fond (180) est alimenté à une colonne de produit de styrène (D4) où le monomère de styrène est obtenu au sommet (190), et un produit lourd en bas (200) ;
j) alimenter les flux de produit de fond lourd (140 et 200), résultant de la seconde colonne de distillation et de la colonne de distillation supplémentaire (D2, D2') et de la colonne de produit de styrène (D4), à la colonne de distillation (D5) ; le flux de produit distillé (220) ainsi obtenu est alimenté conjointement avec le flux contenant de l'éthylbenzène (170) à l'unité d'hydrotraitement (HP) de l'étape c).

2. Le procédé selon la revendication 1, dans lequel le premier flux latéral fractionné (30) obtenu à la suite de l'option b1) ou de l'option b2), comprend une teneur en benzène de 0 à 250 ppmw.

3. Le procédé selon l'une quelconque des revendications 1 à 2, dans lequel le premier flux latéral fractionné (30) obtenu à la suite de l'option b1) ou de l'option b2), comprend une teneur en styrène et en éthylbenzène de 89 % en poids à 99 % en poids par rapport au poids total du flux.

4. Le procédé selon l'une quelconque des revendications 1 à 3, dans lequel le premier flux latéral d'huile de pyrolyse fractionnée (30) obtenu à la suite de l'option b1) ou de l'option b2) comprend de 0,01 à 1 mg de KOH/g de composés à teneur acide mesuré par la méthode d'essai standard ASTM D664.

5. Le procédé selon l'une quelconque des revendications 1 à 4, dans lequel le premier flux latéral fractionné (30) obtenu à la suite de l'option b1) ou de l'option b2), comprend
i. de 89 à 99 % en poids de monomère de styrène plus éthylbenzène, préférablement de 90 à 98 % en poids, plus préférablement de 93 à 95 % en poids ;
ii. de 0 à 500 ppm en poids de benzène, préférablement de 50 à 300 ppm en poids, plus préférablement de 100 à 250 ppm en poids ;
iii. de 0 à 1 % en poids de toluène, préférablement de 0,01 à 0,5 % en poids, plus préférablement de 0,1 à 0,3 % en poids ;
iv. de 0 à 2,5 % en poids de xylènes, préférablement de 0,1 à 2 % en poids, plus préférablement de 0,5 à 1,5 % en poids ;
v. de 0 à 1,5 % en poids de composés aromatiques en C9, autres que l'alpha-méthylstyrène, d'un mélange de cumène ; propénylbenzène, n-propylbenzène ; allyl-benzène, éthyl-toluène et méthylstyrène, préférablement de 0,01 à 1 % en poids, plus préférablement de 0,1 à 0,8 % en poids ;
vi. de 0 à 5 % en poids d'alpha-méthylstyrène, préférablement de 0,1 à 3 % en poids, plus préférablement de 1 à 2,5 % en poids ;
vii. de 0 à 1,5 % en poids de phénylacétylène, préférablement de 0,0001 à 1 % en poids, plus préférablement de 0,001 à 0,5 % en poids ;
viii. de 0 à 2500 ppm en poids de composés hétéroatomiques, préférablement de 1 à 1250 ppm en poids, plus préférablement de 10 à 250 ppm en poids ;
ix. de 0 à 0,1 % en poids de composés aromatiques en C10, d'un mélange de divinylbenzène et éthylstyrène, préférablement de 0,001 à 0,08 % en poids, plus préférablement de 0,01 à 0,05 % en poids ;
x. de 0 à 1 % en poids d'un ou de plusieurs composés choisis dans le groupe constitué par un mélange d'alcanes en C3-C8, d'alcènes en C2-C8, de cycloalcanes en C5-C8 et de cycloalcènes en C5-C8, préférablement de 0,001 à 0,85 % en poids, plus préférablement de 0,01 à 0,5 % en poids ;
xi. de 0 à 0,1 % en poids d'une fraction d'huile lourde, préférablement de 0,0001 à 0,05 % en poids, plus préférablement de 0,001 à 0,01 % en poids ;
xii. de 0 à 50 ppm en poids de métaux, de composés à teneur métallique, préférablement de 0,01 à 25 ppm en poids, plus préférablement de 0,1 à 10 ppm en poids ;
xiii. de 0,5 à 1500 ppm en poids d'hétéroatome, de composés hétéroatomiques, préférablement de 1 à 500 ppm en poids, plus préférablement de 5 à 100 ppm en poids ; et
xiv. de 0 à 120 ppm en poids d'eau, préférablement de 0,1 à 25 ppm en poids, plus préférablement de 1 à 10 ppm en poids.

6. Le procédé selon l'une quelconque des revendications 1 à 5, dans lequel, lorsqu'il suit soit l'option b.1) ou la b.2), avant l'étape c), le procédé comprend en outre un lavage alcalin ou aqueux du premier flux latéral d'huile de pyrolyse fractionnée (30).

7. Le procédé selon l'une quelconque des revendications 1 à 6, dans lequel le premier flux latéral d'huile de pyrolyse fractionnée (30) obtenu en b.1) ou b.2) est introduit dans une unité d'hydrotraitement (HP) comprenant un catalyseur d'hydrotraitement hétérogène en présence d'hydrogène selon l'étape c.1) ; et dans lequel l'unité d'hydrotraitement (HP) est choisie parmi
c.1A) une unité d'hydrotraitement dédiée (HP2) ;
et
c.1B) une unité d'hydrotraitement (HP1) utilisée dans une étape d'hydrogénation d'acétophénone, dans laquelle le premier flux latéral fractionné (30) est introduit dans l'unité d'hydrotraitement (HP1) avec le flux de produit distillé d'une colonne de distillation (D5).

8. Le procédé selon la revendication 7, dans lequel l'unité d'hydrotraitement (HP) de l'étape c.1B) comprend deux ou plusieurs réacteurs d'hydrogénation de boue (HP1₁, HP1₂,...HP1ₙ) connectés en série les uns avec les autres ; dans lequel le premier flux latéral fractionné (30) est réparti entre les différents réacteurs HP1ₙ ; et dans lequel la partie du premier flux latéral fractionné (30) alimentée dans le premier réacteur d'hydrotraitement (HP1₁), donne un flux de produit contenant de l'éthylbenzène qui est alimenté dans le réacteur immédiatement suivant, puis cette action est répétée pour un certain nombre de n réacteurs jusqu'au nième réacteur d'hydrotraitement (HP1ₙ), pour donner un flux de produit contenant de l'éthylbenzène (270).

9. Le procédé selon la revendication 8, dans lequel le premier flux latéral fractionné (30) est réparti entre les différents réacteurs HP1ₙ à une concentration en monomère de styrène inférieure à 15 % en poids sur la base du flux d'alimentation total de chaque réacteur.

10. Le procédé selon l'une quelconque des revendications 1 à 6, dans lequel dans l'étape c), le premier flux latéral fractionné (30) est alimenté à une colonne de distillation (D5) avec les flux de produits de fond lourds (140) et (200), obtenant un courant de produit distillé (220) ; où le courant de produit distillé (220) est alimenté à une unité d'hydrotraitement (HP1) utilisée dans une étape d'hydrogénation d'acétophénone.

11. Le procédé selon l'une quelconque des revendications 1 à 10, qui comprend en outre la conversion d'un résidu lourd contenant des oligomères de styrène (155), qui est obtenu dans l'unité de déshydratation (DH1), dans une unité de pyrolyse, en un flux d'huile de pyrolyse recyclée (10') ; et l'introduction du flux d'huile de pyrolyse recyclée (10') ainsi obtenu dans la première unité de distillation fractionnée (T1) avec le flux liquide d'hydrocarbures résultant de la pyrolyse de déchets de plastique (10) ou, en variante, dans la colonne de distillation (D5).

12. Le procédé selon l'une quelconque des revendications 1 à 11, dans lequel le flux d'éthylbenzène distillé (110) obtenu à l'étape d) comprend
de 93 à 99 % en poids d'éthylbenzène, préférablement de 95 à 99 % en poids ;
de 0,001 à 0,1 % en poids de benzène, préférablement de 0,01 à 0,05 % en poids ;
de 0,01 à 1 % en poids de toluène, préférablement de 0,1 à 0,5 % en poids ;
de 0,01 à 1 % en poids d'o-xylène, préférablement de 0,1 à 0,5 % en poids ;
de 0,01 à 3 % en poids de m-xylène, préférablement de 0,1 à 1,5 % en poids ;
de 0,01 à 3 % en poids de p-xylène, préférablement de 0,1 à 1,5 % en poids ;
de 0,01 à 1 % en poids d'isopropylbenzène, préférablement de 0,1 à 0,5 % en poids ;
de 1 à 1000 ppm en poids de diéthylbenzène, préférablement de 10 à 100 ppm en poids ;
de 0,0001 à 0,1 % en poids de n-propylbenzène, préférablement de 0,001 à 0,01 % en poids ;
de 0,001 à 0,1 % en poids d'un ou de plusieurs composés choisis dans le groupe constitué par un mélange d'alcanes en C3-C8, d'alcènes en C2-C8, de cycloalcanes en C5-C8 et de cycloalcènes en C5-C8, préférablement de 0,01 à 0,06 % en poids ;
de 0,01 à 50 ppmw de composés hétéroatomiques, préférablement de 0,1 à 5 ppmw ; et
de 0,1 à 250 ppmw d'eau, préférablement de 0,1 à 100 ppmw.
